# EUROPEAN PATENT APPLICATION

(11) **EP 4 534 116 A1**
(43) Date of publication of application: **09.04.2025**
(21) Application number: 23831421.5
(22) Date of filing: 27.06.2023
(51) Int. Cl.: A61L 27/24, A61L 27/36, G01N 33/50

(54) **COLLAGEN-RICH ORGANIC COMPOSITION AND PRODUCTION METHOD THEREOF**

(30) Priority: 27.06.2022 JP 2022103089
(71) Applicant: Devine Inc., Sapporo-shi, Hokkaido 060-0807 (JP)
(72) Inventor: OKUBO Naoto, Sapporo-shi, Hokkaido 060-0808 (JP); HOSONO Hidetaka, Sapporo-shi, Hokkaido 060-0807 (JP)
(74) Representative: Prüfer & Partner mbB Patentanwälte · Rechtsanwälte
(86) International application number: PCT/JP2023/023721
(87) International publication number: WO 2024/004981

(57) **Abstract**

[Problem] To provide: a collagen-rich organic composition; a biomaterial using the collagen-rich organic composition for an ingredient material; a biomaterial or other product containing collagen as a main ingredient; a method of producing the collagen-rich organic composition; a method of discerning teeth or bones extracted from a mammal to be used as an ingredient material of a biomaterial or a biomaterial or other product containing collagen as a main ingredient mammal; and a method of using, as an indicator for assessing a potential morbidity risk of a disease in a mammal and/or whether a mammal is potentially in good health or not, PYD as well as PYD and PpP.

[Solution] PYD>100 as well as PYD>100 and PpP>740.

## Description

### [Technical Field]

The present invention relates to a collagen-rich organic composition produced from teeth or bones extracted from a mammal; a biomaterial using the collagen-rich organic composition as an ingredient material; a biomaterial or other product containing collagen as a main ingredient; a method of producing the collagen-rich organic composition; a method of discerning teeth or bones extracted from a mammal to be used as an ingredient material of a biomaterial or a biomaterial or other product containing collagen as a main ingredient mammal; and a method of using, as an indicator for assessing a potential morbidity risk of a disease in a mammal and/or whether a mammal is potentially in good health or not, a quantification value of pyridinoline content (ng/mg) of collagen contained in teeth of a mammal, or a quantification value of pyridinoline content (ng/mg) of collagen contained in teeth of a mammal as well as a weight ratio (pyridinoline content (ng/mg)/ pentosidine content (ng/mg)) calculated from a quantification value of pyridinoline content (ng/mg) of collagen contained in teeth of the mammal and a quantification value of pentosidine content (ng/mg) thereof.

### [Background Art]

Until now, various forms of collagens (commonly referred to simply as "collagen(s)" although "collagen-rich organic composition" is actually a more accurate term) have been developed. Almost all of them are an atelocollagen extracted from a soft tissue within a living organism by means of an enzyme treatment method, which is a collagen wherein a telopeptide present at both of an N terminus and a C terminus in a collagen amino acid sequence is severed and a hardly-soluble collagen fiber is simultaneously removed through a purification process. Additionally, as demineralized freeze-dried bone allograft (DFDBA), demineralized dentin matrix (DDM), and so on, collagens produced by being extracted from hard tissues such as bones and teeth, have been also developed, such collagens are produced through demineralization treatment and mainly used for biomaterials.

Atelocollagens are valued for their collagen quality on such a ground that telopeptides said to have antigenicity have been severed through enzyme treatment whereas undenatured collagens (collagens having a triple helix structure due to having telopeptides retained) derived from soft tissues are valued for their collagen quality on a basis of how collagens high in purity have been produced by means of setting conditions of purification treatment, such as pH. By the way, since on an occasion of producing collagens from hard tissues, the collagens need to be extracted by removing minerals through demineralization treatment, namely, acid treatment, a yield of collagens is restricted and further, extraction thereof takes labor. Therefore, almost of all collagen products being developed turn out to be soft tissue-derived atelocollagens, undenatured collagens, or products utilizing those.

Almost all collagens contained in living tissues are type I collagens. Among the type I collagens, physiologically-crosslinked ones and nonphysiologically-crosslinked ones are present, and while a pyridinoline crosslinking molecule is known as a physiologically-crosslinking molecule, a crosslinking molecule composed of advanced glycation end-products (AGEs) represented by pentosidine is known as a nonphysiologically-crosslinking molecule (Non-patent Literature Document 1). Since pyridinoline crosslinking is a physiological/ enzymatic crosslinking by action of lysyl oxidase and is a regular crosslinking molecule at a specific site in an amino acid sequence of collagen, it contributes to enhancing elasticity and strength of collagen fibers. On the other hand, since pentosidine crosslinking is a nonphysiological/ nonenzymatic crosslinking (aging crosslinking (AGEs crosslinking)) related to by blood glucose and is a crosslinking molecule formed between arginine and lysine residues scattered in an amino acid sequence of collagen due to blood glucose being warmed by body temperature, it is a crosslinking molecule randomly formed at arginine and lysine residues close to each other in an amino acid sequence of collagen and is a factor that disturbs a physiological steric structure of collagen, causing collagen fibers to be lowered in elasticity and strength. Since atelocollagen has, as described above, its telopeptides severed through enzyme treatment, it is a collagen not only containing almost no pyridinoline crosslinking molecules but also containing almost no pentosidine crosslinking molecules because it has its hardly-soluble collagen fibers caused by pentosidine crosslinking, removed through a purification process.

Until now, it has been known that collagen increases in strength and elasticity by pyridinoline crosslinking molecules being increasing in the collagen (Non-patent Literature Document 2), and it has been also known that pentosidine crosslinking is an aging crosslinking (AGEs crosslinking) (Non-patent Literature Document 3). Additionally, it has been reported that pentosidine quantity per unit collagen in human articular cartilage increases linearly with age, and also that a ratio of the pentosidine quantity and pyridinoline quantity per unit collagen (pentosidine content/ pyridinoline content) increases more rapidly with age (Non-patent Literature Documents 4 and 5), and it has been reported that a ratio of the pentosidine quantity and pyridinoline quantity per unit collagen (pentosidine content/ pyridinoline content) increases significantly in noncalcified lesions of dystrophic aortic calcification (Non-patent Literature Document 6).

### [Prior Art Documents]

### [Non-patent Literature]

[Non-patent Literature Document 1] M. Saito, CLINICIAN Vol.554, 1141-1146, 2006
[Non-patent Literature Document 2] M. Saito, THE BONE Vol.21, No.1, 53-58, 2007
[Non-patent Literature Document 3] Sell, D.R.et al., V.M., J. Biol. Chem. Vol.284, 21597-21602,1989
[Non-patent Literature Document 4] A. Uchiyama et al., J. Biochem. Vol.110, 714-718, 1991
[Non-patent Literature Document 5] M. TAKAHASHI et al., Arthritis Rheum. Vol.37, No.5, 724-728, May 1994.
[Non-patent Literature Document 6] H. HOSHI NO et al., Atherosclerosis Vol.112, 39-46, 1995

### [Summary of Invention]

### [Technical Problem]

Since evaluation on quality of the above-mentioned collagen (collagen-rich organic composition) is, at large, made in accordance with what collagen in the collagen-rich organic composition is in degree of purity and origin and is not made on a basis of some molecule-level index of collagen, namely, some amino acid sequence-level index of collagen, it is hard to say that adequate evaluation has been made on quality of collagen in itself, and furthermore, no collagen quality evaluation has been made on a basis of pyridinoline content, which contributes to enhancing collagen quality such as enhancing elasticity and strength of collagen fibers, pentosidine content, which is a factor that disturbs a physiological steric structure of collagen, causing collagen fibers to be lowered in elasticity and strength, and ultimately to contribute to lowering collagen in quality, or on a basis of pentosidine content as well as a weight ratio of pyridinoline content and pentosidine content.

Additionally, not only a collagen-rich organic composition or a biomaterial containing collagen as a main ingredient, which is, on a basis of some molecule-level index of collagen, namely, some amino acid sequence-level index of collagen, evaluated as containing collagen intrinsically good in quality, have not been found, but also as for hard tissue-derived collagen as described above which has its telopeptides remaining, whose collagen yield is restricted, and further of which extraction takes labor, especially teeth, not easy in collagen extraction and restricted in collagen yield, and bones taking a lot of labor for extracting collage because of containing a large amount of lipid, no attempt has been made to provide a collagen-rich organic composition or a biomaterial containing collagen as a main ingredient, which can, on a basis of some molecule-level index of collagen, namely, some amino acid sequence-level index of collagen, be evaluated as containing collagen intrinsically good in quality.

By the way, since a telopeptide region of collagen is believed to show high antigenicity, some collagen products are obtained by using atelocollagen, which has its telopeptides removed through enzyme treatment, but pyridinoline crosslinking molecules are lost by the telopeptides being removed so that suppleness and toughness peculiar to collagen is lost. Further, no manifest evidence exists to show that removing telopeptides reduces antigenicity, and accordingly, removing telopeptides in medical application of collagen has no advantages with regard to immunogenicity, and on another side, proportion of humans who have shown allergy to type I collagen derived from bovines is 2~4%, which can be said to be low as compared with incidence 10~15% of nickel hypersensitivity and about 6% being incidence of latex allergy (Lynn AK et al., J Biomed Mater Res B Appl Biomater. 2004 Nov.15; 71(2): 343-354). The type I collagen is reported to cause no immunological reaction and reported to show no antigenicity (Courtenay JS et al., Nature, 283, 14, 1980, 666-668; David E et al., J. Exp. Med. 1977, 46; 857-868).

Additionally, certainly in Non-patent literature documents 1~6 above, pyridinoline crosslinking molecules and pentosidine crosslinking molecules in collagen are variously mentioned, and besides, ratios of pentosidine quantity and pyridinoline quantity per unit collagen (pentosidine content/ pyridinoline content) in diseases is considered, but none of the literature documents is not in particular about teeth-derived collagen, wherein collagen cannot be easily extracted. In addition, although pyridinoline crosslinking molecules contained in collagen contained and pentosidine crosslinking molecules they entail tends to be in a large amount in tissues physiologically requiring strength such as hard tissues like bones and cartilages and soft tissues like tendons and ligaments (M. TAKAHASHI et al., Anal Biochem Vol. 232, 158-62, 1995), those tissues are tissues to be metabolized, and therefore, pyridinoline crosslinking molecules and pentosidine crosslinking molecules contained therein are also metabolized so that it cannot be said that accumulation amount of pyridinoline crosslinking molecules or pentosidine crosslinking molecules for one life of a host being a mammal is reflected. Since teeth being hard tissues are, contrary thereto, sole tissues not embedded in metabolic cycles among hard tissues, neither pyridinoline crosslinking molecules nor pentosidine crosslinking molecules contained in that collagen contained are metabolized, and therefore, it can be said that accumulation amount of pyridinoline crosslinking molecules and pentosidine crosslinking molecules for almost one life of a host being a mammal is reflected. Accordingly, it is clear that for accurately assessing a potential morbidity risk of a disease in a mammal and/or whether a mammal is potentially in good health or not, teeth-derived collagen (a collagen-rich organic composition), wherein pyridinoline crosslinking molecules and pentosidine crosslinking molecules for one life of a host being a mammal accumulate, should be made an index. Furthermore, since a collagen-rich organic composition or a biomaterial containing collagen as a main ingredient, according to the present application invention, is, under a condition of temperature higher than normal temperature, given strong alkali treatment normally not given to protein containing collagen and is produced through demineralization treatment, namely, acid treatment, it has its endotoxin contained substantially reduced in quantity, and is substantially free of endotoxin or free of endotoxin, or has its endotoxin contained substantially inactivated or such endotoxin inactivated, but the Non-patent literature documents 1~6 above do not, needless to say describing that, even suggest it.

The present invention is intended to provide, by quantifying, using a specified means, pyridinoline content (ng/mg) and pentosidine content (ng/mg) of collagen and calculating, from a PYD being a quantification value of the pyridinoline content (ng/mg) and a PEN being a quantification value of the pentosidine content (ng/mg) which are obtained by those being quantified, a PpP (pyridinoline content (ng/mg)/ pentosidine content (ng/mg)) being a value of a weight ratio, a collagen-rich organic composition which has the PYD or the PYD and the PpP specified and is produced from teeth or bones extracted from a mammal, a biomaterial or a biomaterial or other product containing collagen as a main ingredient, which makes the collagen-rich organic composition an ingredient material, as well as to provide a method of producing the collagen-rich organic composition, a method of discerning teeth or bones to be an ingredient material of a biomaterial or a biomaterial or other product containing collagen as a main ingredient mammal and being extracted from a mammal, which has, when the PYD or the PYD and the PpP is a specified value, a step of selecting the teeth or bones for an ingredient material of a biomaterial or a biomaterial or other product containing collagen as a main ingredient, and a method of using, as an indicator for assessing a potential morbidity risk of a disease in a mammal and/or whether a mammal is potentially in good health or not, a quantification value of pyridinoline content (ng/mg) of collagen contained in teeth of a mammal, or a quantification value of pyridinoline content (ng/mg) of collagen contained in teeth of a mammal as well as a weight ratio (pyridinoline content (ng/mg)/ pentosidine content (ng/mg)) calculated from a quantification value of pyridinoline content (ng/mg) of collagen contained in teeth of the mammal and a quantification value of pentosidine content (ng/mg) thereof, which has a step of obtaining a PYD being a quantification value of pyridinoline content (ng/mg) of collagen contained in teeth of a mammal to be assessed for a potential morbidity risk of a disease and/or whether a mammal is potentially in good health or not and a PEN being a quantification value of pentosidine content (ng/mg) thereof and calculating, from the PYD obtained and the PEN obtained, a PpP (pyridinoline content (ng/mg)/ pentosidine content (ng/mg)) being a value of a weight ratio, a step of obtaining a R-PYD being a quantification value of pyridinoline content (ng/mg) to be a reference of collagen contained in teeth of 1 or 2 or more mammals selected from a mammal not affected by a disease, a mammal affected by a disease, a mammal in good health, and a mammal not in good health and a R-PEN being a quantification value of pentosidine content (ng/mg) to be a reference thereof and calculating, from the R-PYD obtained and the R-PEN obtained, a R-PpP (pyridinoline content (ng/mg)/ pentosidine content (ng/mg)) being a weight ratio to be a reference, and a step of comparing the PYD and the R-PYD or of comparing the PYD and the R-PYD as well as the PpP and the R-PpP.

### [Solution to Problem]

Inventors of the present invention have found out, as a result of extensive research to solve the above problems, that a collagen-rich organic composition obtained by giving strong alkali treatment under a condition of temperature higher than normal temperature and lower than water boiling point to a pulverization product of teeth or bones extracted from a mammal, and giving demineralization treatment under a negative pressure condition to the pulverization product given strong alkali treatment, and a produced biomaterial and a biomaterial or other product containing collagen as a main ingredient are, when a PYD being a quantification value of pyridinoline content (ng/mg) per unit collagen and a PpP (pyridinoline content (ng/mg)/ pentosidine content (ng/mg)) being a value of a weight ratio calculated from pyridinoline content (ng/mg) and pentosidine content (ng/mg) per unit collagen are specified values, of excellent quality in such a manner as collagen contained having a steric structure giving enhanced elasticity and strength, that when the PYD or the PYD and the PpP is a specified value, teeth or bones extracted from a mammal and to be an ingredient material of a biomaterial or a biomaterial or other product containing collagen as a main ingredient can be discerned, and that by comparing, between collagen contained in teeth of a mammal to be assessed for a potential morbidity risk of a disease and/or whether a mammal is potentially in good health or not and collagen contained in teeth of 1 or 2 or more mammals selected from a mammal not affected by a disease, a mammal affected by a disease, a mammal in good health, and a mammal not in good health, the above-mentioned PYD or PYD and PpP for each of them, a potential morbidity risk of a disease in a mammal and/or whether a mammal is potentially in good health or not can be assessed, and have accomplished each of the inventions below.

(1) A collagen-rich organic composition produced from teeth or bones extracted from a mammal, of which (i) or (i) and (ii) below holds:
   (i) As for a PYD being a quantification value of pyridinoline content (ng/mg) of collagen which is quantified with a high-performance liquid chromatography with a fluorescence detector (HPLC-Flu) using heptafluorobutyric acid and formic acid as an ion-pairing reagent and is contained in the collagen-rich organic composition, PYD>100,
   (ii) As for a PpP (pyridinoline content (ng/mg)/ pentosidine content (ng/mg)) being a value of a weight ratio between a PYD being a quantification value of pyridinoline content (ng/mg) and a PEN being a quantification value of pentosidine content (ng/mg), of collagen which is quantified with a high-performance liquid chromatography with a fluorescence detector (HPLC-Flu) using heptafluorobutyric acid and formic acid as an ion-pairing reagent and is contained in the collagen-rich organic composition, PpP>740.
(2) A collagen-rich organic composition according to (1), which is substantially free of endotoxin or free of endotoxin, or alternatively has its endotoxin contained substantially inactivated or such endotoxin inactivated.
(3) A collagen-rich organic composition according to (1), wherein the mammals are 1 or 2 or more mammals to be selected from a group consisting of bovines, swine, horses, sheep, deer, dogs, cats, and humans.
(4) A biomaterial or a biomaterial or other product containing collagen as a main ingredient, which uses a collagen-rich organic composition according to one of (1) to (3) for an ingredient material.
(5) A method of producing a collagen-rich organic composition according to one of (1) to (3), which has
   a step of obtaining a pulverization product by pulverizing teeth or bones extracted from a mammal,
   a step of giving strong alkali treatment to the pulverization product obtained,
   and a step of giving demineralization treatment under a negative pressure condition to the pulverization product given strong alkali treatment.
(6) A production method according to (5), wherein the step of giving strong alkali treatment to the pulverization product obtained is a step of giving strong alkali treatment under a condition of temperature higher than normal temperature and lower than water boiling point to the pulverization product obtained.
(7) A method of discerning teeth or bones extracted from a mammal to be used as an ingredient material of a biomaterial or a biomaterial or other product containing collagen as a main ingredient mammal, which has
   a step of quantifying pyridinoline content (ng/mg) of collagen contained in teeth or bones extracted from a mammal,
   a step of quantifying pentosidine content (ng/mg) of the collagen contained in teeth or bones extracted from a mammal,
   a step of calculating, from a PYD being a value of the pyridinoline content (ng/mg) quantified and a PEN being a value of the pentosidine content (ng/mg) quantified, a PpP (pyridinoline content (ng/mg)/ pentosidine content (ng/mg)) being a weight ratio,
   and a step of selecting, when a PYD being a value of the pyridinoline content (ng/mg) quantified or a PYD being a value of the pyridinoline content (ng/mg) quantified and a PpP (pyridinoline content (ng/mg)/ pentosidine content (ng/mg)) being a value of the weight ratio calculated are specified values, the teeth or bones extracted from a mammal for an ingredient material of a biomaterial or a biomaterial or other product containing collagen as a main ingredient.
(8) A method of discerning teeth or bones extracted from a mammal to be used as an ingredient material of a biomaterial or a biomaterial or other product containing collagen as a main ingredient mammal, which has
   a step of obtaining a pulverization product by pulverizing teeth or bones extracted from a mammal,
   a step of giving strong alkali treatment to the pulverization product obtained,
   a step of giving demineralization treatment under a negative pressure condition to the pulverization product given strong alkali treatment, to obtain a collagen-rich organic composition,
   a step of quantifying pyridinoline content (ng/mg) of collagen contained in the collagen-rich organic composition obtained,
   a step of quantifying pentosidine content (ng/mg) of collagen contained in the collagen-rich organic composition obtained,
   a step of calculating, from a PYD being a value of the pyridinoline content (ng/mg) quantified and a PEN being a value of the pentosidine content (ng/mg) quantified, a PpP (pyridinoline content (ng/mg)/ pentosidine content (ng/mg)) being a value of a weight ratio,
   and a step of selecting, when a PYD being a value of the pyridinoline content (ng/mg) quantified or a PYD being a value of the pyridinoline content (ng/mg) quantified and a PpP (pyridinoline content (ng/mg)/ pentosidine content (ng/mg)) being a value of the weight ratio calculated are specified values, the teeth or bones extracted from a mammal for an ingredient material of a biomaterial or a biomaterial or other product containing collagen as a main ingredient.
(9) A method according to (7) or (8), wherein the step of quantifying pyridinoline content (ng/mg) of collagen contained in the collagen-rich organic composition obtained and the step of quantifying pentosidine content (ng/mg) of collagen contained in the collagen-rich organic composition obtained are a step of quantifying, with a high-performance liquid chromatography with a fluorescence detector (HPLC-Flu) using heptafluorobutyric acid and formic acid as an ion-pairing reagent, pyridinoline content (ng/mg) of collagen contained in the collagen-rich organic composition obtained and a step of quantifying, therewith, pentosidine content (ng/mg) of collagen contained in the collagen-rich organic composition obtained, and wherein as for specified values, PYD>100 or PYD>100 and PpP>740, respectively.
(10) A method of using, as an indicator for assessing a potential morbidity risk of a disease in a mammal and/or whether a mammal is potentially in good health or not, a quantification value of pyridinoline content of collagen contained in teeth of a mammal, or a quantification value of pyridinoline content of collagen contained in teeth of a mammal as well as a weight ratio calculated from a quantification value of pyridinoline content of collagen contained in teeth of the mammal and a quantification value of pentosidine content thereof, which includes
   a step of obtaining, by quantifying pyridinoline content (ng/mg) of collagen contained in teeth of a mammal to be assessed for a potential morbidity risk of a disease and/or whether a mammal is potentially in good health or not, a PYD being a quantification value,
   a step of obtaining, by quantifying pentosidine content (ng/mg) of the collagen contained in teeth of a mammal to be assessed, a PEN being a quantification value,
   a step of calculating, from the PYD obtained and the PEN obtained, a PpP (pyridinoline content (ng/mg)/ pentosidine content (ng/mg)) being a value of a weight ratio,
   a step of obtaining, by quantifying pyridinoline content (ng/mg) of collagen contained in teeth of 1 or 2 or more mammals selected from a mammal not affected by a disease, a mammal affected by a disease, a mammal in good health, and a mammal not in good health, a R-PYD being a reference quantification value,
   a step of obtaining, by quantifying pentosidine content (ng/mg) of collagen contained in teeth of 1 or 2 or more mammals selected from a mammal not affected by a disease, a mammal affected by a disease, a mammal in good health, and a mammal not in good health, a R-PEN being a reference quantification value,
   a step of calculating, from the R-PYD obtained and the R-PEN obtained, a R-PpP (pyridinoline content (ng/mg)/ pentosidine content (ng/mg)) being a reference value of a weight ratio,
   and a step of comparing the PYD and the R-PYD or comparing the PYD and the R-PYD as well as the PpP and the R-PpP.
(11) A method according to (10) wherein the step of obtaining, by quantifying pyridinoline content (ng/mg), a R-PYD being a reference quantification value and the step of obtaining, by quantifying pentosidine content (ng/mg), a R-PEN being a reference quantification value are a step of quantifying, with a high-performance liquid chromatography with a fluorescence detector (HPLC-Flu) using heptafluorobutyric acid and formic acid as an ion-pairing reagent, the pyridinoline content (ng/mg) to obtain a R-PYD being a reference value and a step of quantifying, therewith, the pentosidine content (ng/mg) to obtain a R-PEN being a reference value, and where a step of comparing the PYD and the R-PYD or comparing the PYD and the R-PYD as well as comparing the PpP and the R-PpP is a step of checking whether or not, as for the PYD, PYD>100 or checking whether or not, as for the PYD, PYD>100 and whether or not, as for the PpP, PpP>740.

### [Effect of Invention]

The present invention makes it allowable to provide a collagen-rich organic composition, a biomaterial, and a biomaterial or other product containing collagen as a main ingredient, of excellent quality in such a manner that contained collagen obtained by giving strong alkali treatment to a pulverization product of teeth or bones extracted from a mammal (preferably, giving strong alkali treatment under specified conditions such as a condition of temperature higher than normal temperature) and giving demineralization treatment under a negative pressure condition to the pulverization product given strong alkali treatment has a steric structure giving excellent elasticity and strength, additionally makes it allowable to discern teeth or bones extracted from a mammal and being as an ingredient material of a biomaterial or a biomaterial or other product containing collagen as a main ingredient, and further makes it allowable to use, as an indicator for assessing a potential morbidity risk of a disease in a mammal and/or whether a mammal is potentially in good health or not, a quantification value of pyridinoline content (ng/mg) of collagen contained in teeth of a mammal, or a quantification value of pyridinoline content (ng/mg) of collagen contained in teeth of a mammal as well as a weight ratio (pyridinoline content (ng/mg)/ pentosidine content (ng/mg)) calculated from a quantification value of pyridinoline content (ng/mg) of collagen contained in teeth of the mammal and a quantification value of pentosidine content (ng/mg) thereof.

### [Brief Description of Drawings]

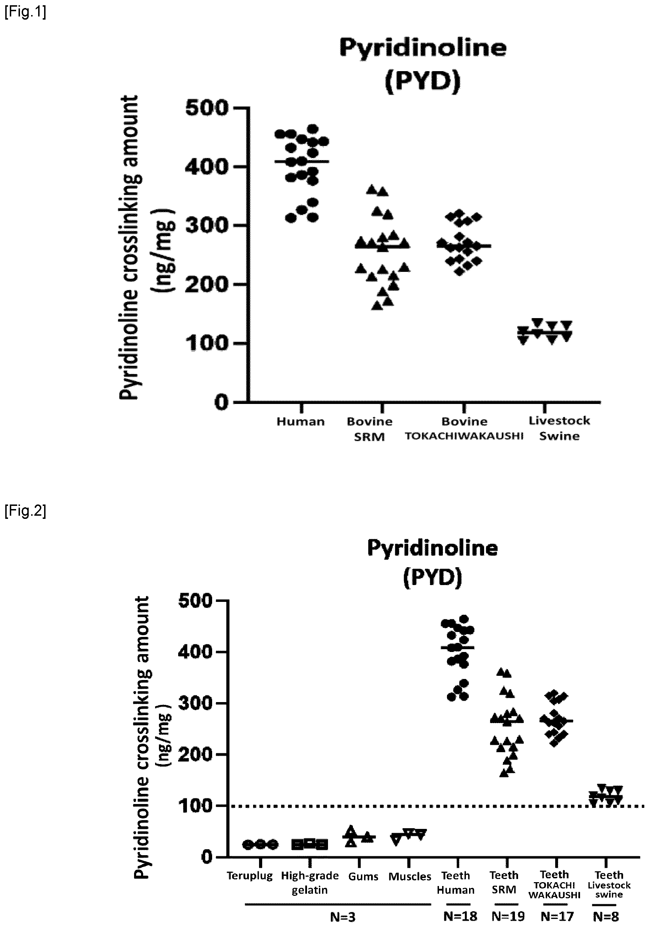

[Fig.1] A graph diagram showing a distribution of a PYD being a quantification value of pyridinoline content (ng/mg) obtained by quantifying, with a high-performance liquid chromatography with a fluorescence detector (HPLC-Flu) using heptafluorobutyric acid and formic acid as an ion-pairing reagent, collagen contained in a collagen-rich organic composition derived from dentin of teeth (62 cases in total) of 18 cases of humans, 36 cases of bovines (therein, 19 cases of bovine specified risk materials (SRM), 17 cases of TOKACHIWAKAUSHI (a male Holstein through early fattening (14 months old) with a Japan-registered trademark)), and 8 cases of livestock swine (six months old).
[Fig.2] A graph diagram showing a distribution of a PYD being a quantification value of pyridinoline content (ng/mg) obtained by quantifying, with a high-performance liquid chromatography with a fluorescence detector (HPLC-Flu) using heptafluorobutyric acid and formic acid as an ion-pairing reagent, collagen (n=62) contained in a collagen-rich organic composition derived from dentin of teeth (62 cases in total) of 18 cases of humans, 36 cases of bovines (therein, 19 cases of bovine SRM, 17 cases of TOKACHIWAKAUSHI (a Japan-registered trademark)), and 8 cases of livestock swine, Teruplug (n=3, bovine dermis-derived atelocollagen, telopeptide not-containing type treatment), high-grade gelatin (n=3, porcine-skin acid treatment, telopeptide preservation type treatment method, low-endotoxin collagen), TOKACHIWAKAWUSHI (a Japan-registered trademark)'s gums (n=3), and TOKACHIWAKAWUSHI (a Japan-registered trademark)'s muscles (n=3). In the figure, a broken line shows a threshold value of a PYD.

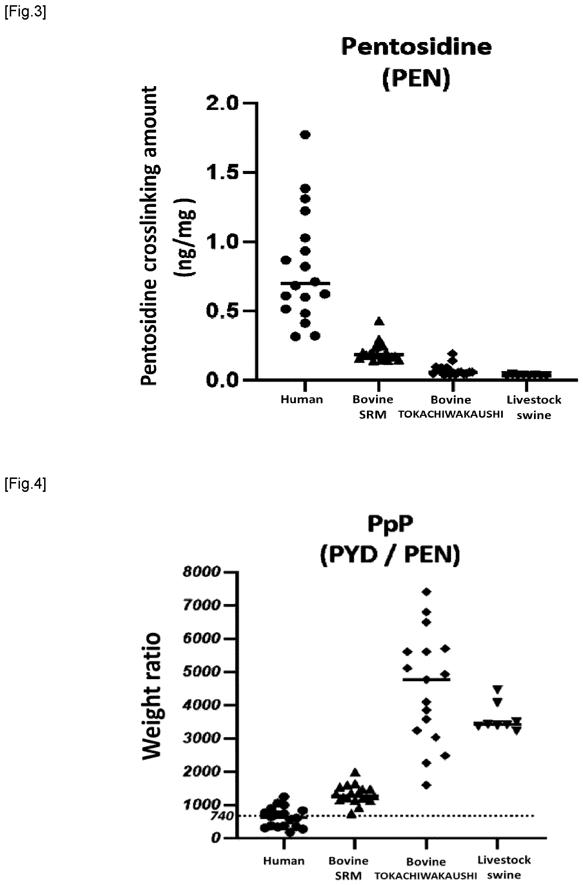

[Fig.3] A graph diagram showing a distribution of a PEN being a quantification value of pentosidine content (ng/mg) obtained by quantifying, with a high-performance liquid chromatography with a fluorescence detector (HPLC-Flu) using heptafluorobutyric acid and formic acid as an ion-pairing reagent, collagen contained in a collagen-rich organic composition derived from dentin of teeth (62 cases in total) of 18 cases of humans, 36 cases of bovines (therein, 19 cases of bovine SRM, 17 cases of TOKACHIWAKAUSHI (a Japan-registered trademark)), and 8 cases of livestock swine.
[Fig.4] A graph diagram showing a value distribution of a PpP (pyridinoline content (ng/mg)/ pentosidine content (ng/mg)) being a value of a weight ratio calculated from a PYD being a quantification value of pyridinoline content (ng/mg) and a PEN being a quantification value of pentosidine content (ng/mg), which are obtained by quantifying, with a high-performance liquid chromatography with a fluorescence detector (HPLC-Flu) using heptafluorobutyric acid and formic acid as an ion-pairing reagent, collagen contained in a collagen-rich organic composition derived from dentin of teeth (62 cases in total) of 18 cases of humans, 36 cases of bovines (therein, 19 cases of bovine SRM, 17 cases of TOKACHIWAKAUSHI (a Japan-registered trademark)), and 8 cases of livestock swine.

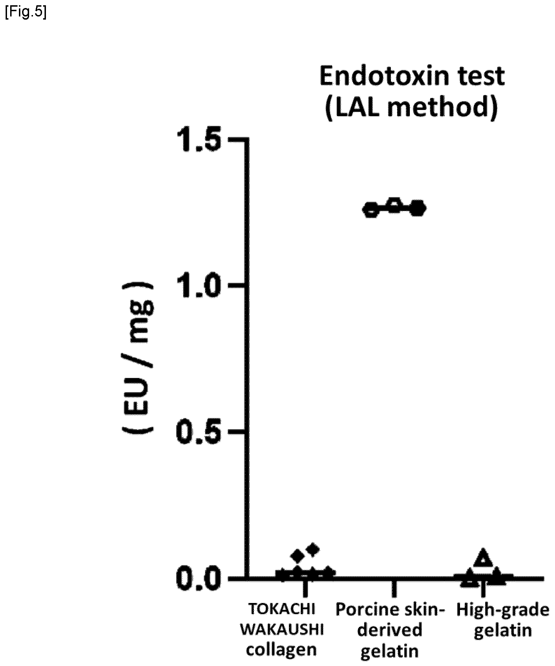

[Fig.5] A graph diagram showing a distribution of an endotoxin value (an endotoxin level; EU/mg) obtained by quantifying, in an endotoxin test (LAL method), collagen contained in a collagen-rich organic composition derived from dentin of teeth of 6 cases of TOKACHIWAKAUSHI (a Japan-registered trademark), 3 cases of porcine skin-derived gelatin, and 3 cases of high-grade gelatin.

### [Description of Embodiments]

As below, a collagen-rich organic composition produced from teeth or bones extracted from a mammal, a biomaterial using the collagen-rich organic composition for an ingredient material, a biomaterial or other product containing collagen as a main ingredient, a method of producing the collagen-rich organic composition, a method of discerning teeth or bones extracted from a mammal to be used as an ingredient material of a biomaterial or a biomaterial or other product containing collagen as a main ingredient, and a method of using, as an indicator for assessing a potential morbidity risk of a disease in a mammal and/or whether a mammal is potentially in good health or not, a quantification value of pyridinoline content (ng/mg) of collagen contained in teeth of a mammal, or a quantification value of pyridinoline content (ng/mg) of collagen contained in teeth of a mammal as well as a weight ratio (pyridinoline content (ng/mg)/ pentosidine content (ng/mg)) calculated from a quantification value of pyridinoline content (ng/mg) of collagen contained in teeth of the mammal and a quantification value of pentosidine content (ng/mg) thereof. A numerical range denoted by using "~" or "from" in the present description means a range including numerical values presented at a top of and a bottom of a "~" or "from" phrase as a lower limit value and an upper limit value.

A collagen-rich organic composition according to the present invention is a collagen-rich organic composition produced from teeth or bones extracted from a mammal, of which (i) or (i) and (ii) below holds.
(i) As for a PYD being a quantification value of pyridinoline content (ng/mg) of collagen which is quantified with a high-performance liquid chromatography with a fluorescence detector (HPLC-Flu) using heptafluorobutyric acid and formic acid as an ion-pairing reagent and is contained in the collagen-rich organic composition, PYD>100,
(ii) As for a value of a PpP (pyridinoline content (ng/mg)/ pentosidine content (ng/mg)) being a value of a weight ratio between a PYD being a quantification value of pyridinoline content (ng/mg) and a PEN being a quantification value of pentosidine content (ng/mg), of collagen which is quantified with a high-performance liquid chromatography with a fluorescence detector (HPLC-Flu) using heptafluorobutyric acid and formic acid as an ion-pairing reagent and is contained in the collagen-rich organic composition, PpP>740.

Although a "mammal" means, basically in the present invention, an animal sexually reproducing, many species currently present of which are viviparous and feed their children with breast milk, and is, for such an animal, not restricted in particular, the animals that can be mentioned as such are, for example, humans; apes such as orangutans, gorillas, chimpanzees, bonobos, and monkeys belonging to gibbon family; large mammals such as monkeys other than those belonging to gibbon family, bovines, horses, swine, wild boars, sheep, goats, antelopes, bears, sea lions, seals, walruses, northern sea lions, fur seals, and whales; small mammals such as dogs, cats, rabbits, rats, squirrels, weasels, raccoons, and mongooses, in which bovines, swine, horses, sheep, deer, dogs, cats, and humans can be mentioned as a favorable mammal, bovines, swine, and horses can be mentioned as a more favorable mammal, and bovines and swine can be mentioned as a most favorable mammal.

"Collagen" is mainly one of proteins making up various organs, blood vessels, nerves, skins, ligaments, tendons, bones, cartilages, dental dentins, and so on of a vertebrate and to be a framework of various tissues within a living organism thereof, and is a main constituent of an extracellular matrix of an multicellular animal. The "collagen" mainly refers to type I collagen in the present invention whereas a collagen-rich organic composition is, on some occasion, referred to simply as "collagen" in the present description. Additionally, since the "collagen" in the present invention is collagen of teeth or bones while the teeth and bones are tissues on which strong load such as body weight and bite force is imposed, the collagen of teeth or bones is required to have strength high enough to support such tissues.

Hard-tissue collagen can, for example, with BMP-2, BMP-4,BMP-7, or osteocalcin being detected, be determined to derive from hard tissues, and with dentin sialoprotein (DSP), dentin glycoprotein (DGP), or dentin phosphoprotein (DPP), be determined to derive from teeth.

A "collagen-rich organic composition" generally refers to an organic composition whose collagen contained accounts for high proportion of a whole organic composition, and atelocollagen, demineralized freeze-dried bone allograft (DFDBA), and demineralized dentin matrix (DDM) fall under the "collagen-rich organic composition" mentioned generally. In the present invention, the "collagen-rich organic composition" refers to, out of a collagen-rich organic composition mentioned generally, an organic composition whose collagen accounts for a proportion of at least 60% or more of the whole organic composition, favorably 70% or more, more favorably 80% or more, further more favorably 85% or more, much further more favorably 90% or more, most favorably 95%. Additionally, the above-mentioned DSP, DGP, and DPP, and so on can be, in the present invention, mentioned as an organic composition contained in a collagen-rich organic composition and other than collagen.

Additionally, a "biomaterial," generally, refers to a material used in a body or used in a state of being contact with a biological constituent such as protein and cells, and a "biomaterial containing collagen as a main ingredient" refers to one containing collagen as a main ingredient in a whole biomaterial, and demineralized freeze-dried bone allograft (DFDBA) and demineralized dentin matrix (DDM) fall under "biomaterial containing collagen as a main ingredient" mentioned generally. In the present invention, the "biomaterial containing collagen as a main ingredient" refers to, out of a biomaterial containing collagen as a main ingredient mentioned generally, a biomaterial containing collagen as a main ingredient, whose collagen accounts for a proportion of at least 40% or more of the whole biomaterial, favorably 45% or more, more favorably 50% or more, further more favorably 55% or more, much further more favorably 60% or more, and most favorably 65%. Additionally, the above-mentioned DSP, DGP, and DPP, and so on can be mentioned as an organic composition contained in a biomaterial and other than collagen.

In the present invention, contained pyridinoline of collagen contained in a collagen-rich organic composition and contained pentosidine thereof are, respectively, a pyridinoline making up a pyridinoline crosslinking molecule being a physiologically-crosslinking molecule in the collagen and a pentosidine making up a pentosidine crosslinking molecule being a nonphysiologically-crosslinking molecule in the collagen. In the present invention, pyridinoline content (ng/mg) of the collagen contained in a collagen-rich organic composition and pentosidine content (ng/mg) of the collagen contained in a collagen-rich organic composition are quantified respectively, and further, a PYD being a quantification value of the pyridinoline content (ng/mg) is divided by a PEN being a quantification value of the pentosidine content (ng/mg), by which a weight ratio (pyridinoline content (ng/mg)/ pentosidine content (ng/mg)) between the PYD being a quantification value of the pyridinoline content (ng/mg) and the PEN being a quantification value of the pentosidine content (ng/mg) is calculated.

A collagen-rich organic composition according to the present invention can, when a PYD or a PYD and a PpP is the above-mentioned specified value, be said to be of excellent quality since collagen contained thereby has enhanced elasticity and strength. Reasons for that are expounded below.

A pyridinoline crosslinking being a physiological/ enzymatic crosslinking molecule is, not too much to say, programmed mainly in order to enhance fundamental strength of hard tissues, and is, as described above, a crosslinking molecule to be, by enzymatic action of lysyl oxidase, formed at two lysine residues in telopeptides present at an N terminus and a C terminus respectively in an amino acid sequence of collagen, and at one lysine residue in an amino acid sequence of collagen except both ends thereof. Since a pyridinoline crosslinking molecule to be formed at two lysine residues in telopeptides present at an N terminus and a C terminus respectively in an amino acid sequence of collagen is a crosslinking molecule between collagen fibers, it can be said to greatly contribute to maintaining a steric structure property of tropocollagen (whole collagen sequence) which is in a triple helix structure.

Therefore, when pyridinoline crosslinking molecules increase in collagen contained in a living tissue, they make quality of collagen contained in that living tissue high in suppleness and consistency to enhance, of the collagen, strength and calcification degree, and can be an element of enhancing activity of cells making up the living tissue, that is, by activity of tissue stem cells involved in tissue repair being elevated, an element of homeostasis of an individual being easily maintained. Therefore, since collagen derived from teeth or bones is, as compared with soft-tissue collagen represented by skins, great in pyridinoline content, the collagen derived from teeth or bones is supple, high in strength, and high in elasticity.

Since as for collagen derived from such pyridinoline crosslinking molecules and contained in a collagen-rich organic composition, namely, collagen to be a raw material of the collagen-rich organic composition and contained in teeth or bones extracted from an mammal, a PYD being a quantification value of pyridinoline content (ng/mg) leads to collagen contained in teeth or bones extracted from a mammal being of more excellent quality as the value is greater, a collagen-rich organic composition produced from teeth or bones extracted from such a mammal can be said to be an excellent organic composition.

A PYD being a quantification value of pyridinoline content (ng/mg) is, as shown in Fig.1, included in a PYD scope according to the present invention in any way for collagen contained in a collagen-rich organic composition derived from dentin of teeth (62 cases in total) being one example of a hard tissue of 18 cases of humans, being a mammal, as well as 36 cases of bovines (therein, 19 cases of bovine SRM (Hayakita Factory, Hokkaido Livestock Corporation), 17 cases of TOKACHIWAKAUSHI (a male Holstein with a Japan-registered trademark and through early fattening (14 months old); JA Tokachi Shimizu)), and 8 cases of livestock swine (six months old; Hayakita Factory, Hokkaido Livestock Corporation), being a mammal and a livestock animal simultaneously. Here, the "SRM" refers to Specified Risk Materials, and in Japan, tonsils and distal ileum (part of small intestines) at all monthly ages, as well as heads (excluding tongue and cheek meat), spine, and spinal cord of animals aged over 30 months are deemed to be such specified risk materials (Food Safety Commission, Cabinet Office).

On the other hand, a PYD being a quantification value of pyridinoline content (ng/mg) is, as shown in Fig.2, 50 or lower in any way for soft tissue-derived Teruplug (n=3, bovine dermis-derived atelocollagen, telopeptide not-containing type treatment; Olympus Terumo Biomaterials Corp.), high-grade gelatin (n=3, porcine-skin acid treatment, telopeptide preservation type treatment method, low-endotoxin collagen; Nippi, Incorporated), TOKACHIWAKAWUSHI (a Japan-registered trademark)'s gums (n=3; JA Tokachi Shimizu), and TOKACHIWAKAWUSHI (a Japan-registered trademark)'s muscles (n=3; JA Tokachi Shimizu).

From the above, a PYD value according to the present invention can be set to PYD>100 in view of a lower limit value of a PYD of collagen contained in a collagen-rich organic composition derived from dentin of teeth being a hard tissue of 18 cases of humans, being a mammal, as well as 36 cases of bovines and 8 cases of livestock swine, being a mammal and a livestock animal simultaneously.

That is, a fact that in the present invention, PYD>100 for a quantification value of pyridinoline content (ng/mg) PYD of collagen contained in a collagen-rich organic composition, namely, collagen used for a raw material of the collagen-rich organic composition and contained in teeth or bones extracted from a mammal leads to a fact that the collagen contained in a collagen-rich organic composition can be presumed not to be, in a mammal, collagen derived from a living tissue excluding teeth or bones.

On the other hand, a crosslinking with advanced glycation end-products (AGEs) represented by pentosidine crosslinking molecules being nonphysiologically crosslinking molecules is a pathological crosslinking (aging crosslinking (AGEs crosslinking)) caused by blood glucose, and is, as described above, a crosslinking molecule formed at arginine and lysine residues scattered in an amino acid sequence of collagen due to blood glucose being warmed by body temperature. Since pentosidine crosslinking molecules are formed nonspecifically (randomly) when arginine and lysine residues in an amino acid sequence of collagen are near to each other, they can put collagen fibers in a state of being entangled, and make the collagen fibers lose an arrangement pattern and are a factor of disturbing a steric structure of tropocollagen, and therefore, pentosidine crosslinking molecules increased in an amino acid sequence of collagen lead to weakening collagen fibers in suppleness, strength, and elasticity. Since formation of this pathological crosslinking molecule is caused by blood glucose, its accumulation amount is said to increase in dependance on a dietary life habit (carbohydrate intake habit) of a host. Additionally, since saccharide matters being a raw material of pentosidine crosslinking molecules are supplied in a transvascular manner, collagen being a supportive basis of tissues of a whole body is evenly crosslinked in a homogeneous manner, regardless of where the tissues lie. That is, excessive intake of sugars can be said to lead to lowering uniformly quality of collagen to be a supportive basis of a body, regardless of whether in hard tissues or soft tissues.

Additionally, when pentosidine crosslinking molecules increase in collagen, that collagen contained in a living tissue has a state like a brittle chalk because the pentosidine crosslinking molecules are glycation crosslinking molecules generated by Maillard reaction so that the collagen contained in a living tissue has its strength and calcification degree lowered. Although a product of a late period of Maillard reaction is said to be advanced glycation end-products (AGEs), pentosidine crosslinking molecules are AGEs and collagen of mammals which are kept constantly in a state of high glucose levels in blood due to their dietary habits high in carbohydrate intake frequency contains a lot of pentosidine crosslinking molecules as compared with collagen of mammals which are at same ages (monthly ages) and whose dietary habits are well-balanced, and therefore, it can be said that aging crosslinking is advanced in collagen of mammals which are constantly in high glucose levels in blood. The pentosidine crosslinking molecules generated disturb a regular arrangement order of collagen fibers to make collagen have its flexibility impaired and change it into hard and brittle one, simultaneously inhibit the collagen from being metabolized, and as a result, lead to quality deterioration of collagen making up a whole body and cause tissues to be lowered in suppleness. Since collagen is a crucial constituent of supportive tissues supporting not only all hard tissues such as bones and teeth but also all soft tissues including nerves and blood vessels, the quality deterioration of collagen can be a factor of making quality of hard tissues/ soft tissues in a body deteriorate. For example, when glycation of bones lowers bone quality, the bones are embrittled, which causes osteoporosis, and besides, when glycation of blood vessel walls lowers quality of blood vessels, arteries are hardened, which generates so-called arteriosclerosis and thereby, brings about a tissue disorder such as blood pressure elevation occurring. As similar matters hold true for "(1) diabetic retinopathy (if exacerbated, blindness)," "(2) diabetic nephropathy (if exacerbated, proceeding to kidney dialysis)," and "(3) diabetic neuropathy (if exacerbated, for example, gangrene of hand fingers and toe fingers due to blood circulation disorder)" called three major complications of diabetes, which are said to be respectively caused by "(1) blood circulation disorder of retinae," "(2) capillary vessel disorder of kidney glomeruli being a blood filtration instrument," and "(3) blood flow disorder of four distal limbs due to neuropathy of capillary vessels, and arteriosclerosis," all of these can be said to be mainly caused by collagen quality having been lowered by glycation.

In addition, when advanced glycation end-products (AGEs) represented by pentosidine crosslinking molecules increase, AGEs can more easily bind to RAGEs had by cells making up tissues of a whole body and being a receptor of AGEs, and as a result, an in vivo environment where their signals can be activated more easily is established. Since this promotes NF-*κ*B signals and so on being related to inflammation, AGEs which are represented by pentosidine crosslinking molecules and induce chronic inflammation result in acting on tissues excessively glycated, as a chronic inflammation triggering factor. They are believed to be, by this mechanism, a factor of exacerbating chronic inflammatory diseases represented by inflammatory bowel diseases (IBDs), collagen diseases, and so on. As described above, collagen glycation leads to a factor of gradually undermining a living body in diverse directions, lowers quality of life (QOL) without being noticed, and can be a major factor of alienating living creatures from healthful longevity. It is reported that both pyridinoline crosslinking molecules and pentosidine crosslinking molecules in collagen contained in human living tissues increases as ages grow (Shimizu, "Age-related nonenzymatic glycation modification of dentin collagen protein," Osaka University Knowledge Archive. 2015; Walters c.et al., Calcif. Tissue. Int. Vol. 35, 401-405, 1983).

As a PEN being a quantification value of pentosidine content (ng/mg) of collagen derived from such pentosidine crosslinking molecules and contained in a collagen-rich organic composition, namely, collagen used for a raw material of the collagen-rich organic composition and contained in teeth or bones extracted from a mammal is a smaller and smaller value, the collagen contained in teeth or bones extracted from a mammal is of more excellent quality, and therefore, a collagen-rich organic composition produced from teeth or bones extracted from such a mammal can be said to be an excellent organic composition.

On another side, since humans have a strong tendency of leading a life while eating miscellaneous stuffs as the grow, they have a high tendency of accumulating pentosidine crosslinking molecules as compared with wholesome livestock animals whose feeds are managed in a predetermined quality throughout lives, and clearly have, therewith, a tendency of having their collagen inferior in quality and such a tendency also appears in Fig.3, and therefore, humans should not, for a standard indicator of pentosidine content (ng/mg), be made to be an indicator, but livestock animals living in healthy standard saccharide intake habits should be made to be an indicator.

Accordingly, a PEN being a quantification value of pentosidine content (ng/mg) of collagen contained in a collagen-rich organic composition derived from dentin of teeth being one example of a hard tissue of 44 cases of livestock animals (therein, 19 cases of bovine SRM (Hayakita Factory, Hokkaido Livestock Corporation), 17 cases of TOKACHIWAKAUSHI (a Japan-registered trademark; JA Tokachi Shimizu), and 8 cases of livestock swine (Hayakita Factory, Hokkaido Livestock Corporation) can be, as shown in Fig.3, set to 0<PEN<0.4 in view of upper limit values of 44 cases of livestock animals.

In the present invention, a "livestock animal" refers to a mammal whose feeds are managed in a predetermined quality, and bovines, swine, horses, sheep, goats, and deer can be mentioned as favorable livestock animals.

Additionally, in the present invention, that a PpP (pyridinoline content (ng/mg)/ pentosidine content (ng/mg)) being a value of a weight ratio between collagen derived from pyridinoline crosslinking molecules and contained in a collagen-rich organic composition, namely, a PYD being a quantification value of pyridinoline content (ng/mg) of teeth or bones used for a raw material of the collagen-rich organic composition and extracted from an mammal, and, collagen derived from pentosidine crosslinking molecules and contained in a collagen-rich organic composition, namely, a PEN being a quantification value of pentosidine content (ng/mg) of teeth or bones used for a raw material of the collagen-rich organic composition and extracted from an mammal allows it to be presumed from the above-mentioned properties of pyridinoline crosslinking and pentosidine crosslinking that collagen contained in the collagen-rich organic composition or a biomaterial containing collagen as a main ingredient is high-quality collagen which is supple, high in strength, and high in elasticity, as well as where arrangement patterns and steric structure properties of collagen fibers are preserved. However, on this occasion, it is presupposed that the PYD is a predetermined value, namely, that collagen contained in the collagen-rich organic composition or a biomaterial containing collagen as a main ingredient is not collagen derived from living tissues other than teeth or bones.

Pyridinoline content (ng/mg) of collagen contained in a collagen-rich organic composition, namely, collagen used for a raw material of the collagen-rich organic composition and contained in teeth or bones extracted from an mammal, and pentosidine content (ng/mg) thereof can be quantified by using a high-performance liquid chromatography with a fluorescence detector (HPLC-Flu) using heptafluorobutyric acid and formic acid as an ion-pairing reagent. Then, since for a collagen-rich organic composition according to the present invention, a PpP (pyridinoline content (ng/mg)/ pentosidine content (ng/mg)) being a value of a weight ratio between a PYD being a quantification value of pyridinoline content (ng/mg) and a PEN being a quantification value of pentosidine content (ng/mg), of collagen which is contained in the collagen-rich organic composition and through a high-performance liquid chromatography with a fluorescence detector (HPLC-Flu) using heptafluorobutyric acid and formic acid as an ion-pairing reagent, makes livestock animals indicators for the PEN, it can be set to be PpP>740, in view of lower limit values of 44 cases of livestock animals, in accordance with Fig.4.

Preparation of a sample to be provided for quantification of the pyridinoline content (ng/mg) and pentosidine content (ng/mg) above can be carried out by using a preparation means publicly-known and conventional in this field, and can be, for example, carried out as follows, generally.

(1) Dried collagen powder produced by a method of producing a collagen-rich organic composition according to the present invention is measured and taken into a test tube, is swollen by means of a small amount of 6N hydrochloric acid under deaeration.
(2) The dried collagen has 6N hydrochloric acid added to, and is, after being deaerated, sealed.
(3) It is hydrolyzed by being given heating treatment at 110°C one day and one night.
(4) A sample is cooled to room temperature.
(5) Surplus hydrochloric acid is removed by being dried under reduced pressure while being warmed, and a collagen hydrolysate is obtained thereby.
(6) Collagen hydrolysate obtained is solubilized in 10% methanol aqueous solution.
(7) Centrifuge at 12000 rpm under room temperature for five minutes and use a supernatant as a sample.
(8) A known amount of a standard substance is added to the sample, and thereby, an extraction rate and a calibration curve are obtained to allow collagen contents to be modified as appropriate.

Quantification of pyridinoline content (ng/mg) and pentosidine content (ng/mg) through a high-performance liquid chromatography with a fluorescence detector (HPLC-Flu) using heptafluorobutyric acid and formic acid as an ion-pairing reagent can also be carried out by using a quantification means publicly-known and conventional in this field, and can be, for example, carried out as follows, generally.

(1) A ultrapure water (mobile phase A) containing 0.05% heptafluorobutyric acid, and a methanol/ethanol 1:1 mixture solution (mobile phase B) containing 0.05% formic acid are used as a mobile phase, and thereby, liquid delivery is carried out in a gradient setting to make detection.
(2) As for a specific liquid delivery condition, the liquid delivery is initiated with a detection column temperature being 40°C and a flow rate being 0.5 mL/min and in a proportion of mobile phase A 90% and mobile phase B 10%, and goes on for 0.5 minutes.
(3) Then, after the mobile phase B is increased to 22% by spending 1 minute and a concentration gradient is applied thereby, the liquid delivery is carried out for 7.5 minutes as it is.
(4) Next, the mobile phase B is increased to 80% by spending 1 minute, and further, the liquid delivery is carried out for 1 minute.
(5) Finally, after the liquid delivery is carried out in a proportion of mobile phase A 10% and mobile phase B 90%, it is equilibrated for 4 minutes in a proportion of mobile phase A 90% and mobile phase B 10%.
(6) (1)~(5) above are carried out for 15 minutes in total for one sample. They use Cadenza CD-C18 with its length 150mm and its inner diameter 3mm for a separation column and use a fluorescent detector for detection. Monitoring is carried out for first-half 8 minutes at 295nm in excitation wavelength and 395nm in emission wavelength, and for second-half 7 minutes at 325nm in excitation wavelength and 385nm in emission wavelength. Such monitoring makes contained pyridinoline be detected at a retention time of about 6.5 minutes or around and contained pentosidine be detected at a retention time of about 9.1 minutes or around.

Next, since a collagen-rich organic composition according to the present application invention is, under a condition of temperature higher than normal temperature, given strong alkali treatment normally not given to protein containing collagen and is produced through demineralization treatment, namely, acid treatment, it has, as shown in Fig.5, its endotoxin contained substantially reduced in quantity, or its endotoxin contained substantially reduced in activity, and therefore, it is substantially free of endotoxin or free of endotoxin, or alternatively has its endotoxin contained substantially inactivated or such endotoxin inactivated.

Quantity and activity "wherein reduction has occurred" are typically "statistically significant" quantity and activity, and include, in a quantity wherein there is no composition (wherein neither drug nor compound is present) or wherein generation is made by a control composition, or in an activity wherein excitation is made by such a control composition, all integers therebetween, and can include decrease by 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95% or 100%.

"Substantially free of endotoxin" usually means that a trace of endotoxin (e.g. quantity where no physiological effect clinically harmful is present against a subject) at most or favorably an undetectable quantity of endotoxin is contained. Although endotoxins are toxins related to specific bacteria, typically gram-negative bacteria, some endotoxins can be found in gram-positive bacteria such as *Listeria monocytogenes.* Endotoxins most crucial are lipopolysaccharides (LPS) or lipooligosaccharides (LOS) found in outer membranes of various gram-negative bacteria and present central pathological features in capabilities of these bacteria to cause diseases.

A small quantity of endotoxin in human bodies can, among other harmful physiological effects, cause pyrexia, blood pressure depression, as well as inflammation and activation of blood coagulation. Accordingly, it is desirable on many occasions to remove almost all or all traces of endotoxins from a collagen-rich organic composition according to the present application invention and a biomaterial containing collagen as a main ingredient, according to the present application invention, which is because a small quantity thereof can adversely affect humans.

Endotoxins can be detected by using techniques publicly-known and conventional in this field. For example, a Limulus Amebocyte Lysate assay (LAL assay), where blood derived from horseshoe crabs is utilized, is a highly sensitive assay for detecting presence of endotoxins. In this test, a very low level of lipopolysaccharide (LPS) can, due to a strong enzymatic cascade which amplifies this reaction, cause a limulus lysate coagulation which can be detected. Such endotoxins can be quantified even by means of an enzyme-linked immunosorbent assay (ELISA).

Due to being substantially free of endotoxin, a protein whose endotoxin level lies at approximately 0.001, 0.002, 0.003, 0.004, 0.005, 0.006, 0.007, 0.008, 0.009, 0.01, 0.011, 0.012, 0.013, 0.014, 0.015, 0.016, 0.017, 0.018, 0.019, 0.02, 0.021, 0.022, 0.023, 0.024, 0.025, 0.026, 0.027, 0.028, 0.029, 0.03, 0.031, 0.032, 0.033, 0.034, 0.035, 0.036, 0.037, 0.038, 0.039, 0.04, 0.041, 0.042, 0.043, 0.044, 0.045, 0.046, 0.047, 0.048, 0.049, 0.05, 0.051, 0.052, 0.053, 0.054, 0.055, 0.056, 0.057, 0.058, 0.059, 0.06, 0.061, 0.062, 0.063, 0.064, 0.065, 0.066, 0.067, 0.068, 0.069, 0.07, 0.071, 0.072, 0.073, 0.074, 0.075, 0.076, 0.077, 0.078, 0.079, 0.08, 0.081, 0.082, 0.083, 0.084, 0.085, 0.086, 0.087, 0.088, 0.089, 0.09, 0.091, 0.092, 0.093, 0.094, 0.095, 0.096, 0.097, 0.098, 0.099, 0.1, 0.11, 0.12, 0.13, 0.14, 0.15, 0.16, 0.17, 0.18, 0.19, 0.2, 0.21, 0.22, 0.23, 0.24, 0.25, 0.26, 0.27, 0.28, 0.29, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, 0.7, 0.71, 0.72, 0.73, 0.74, 0.75, 0.76, 0.77, 0.78, 0.79, 0.8, 0.81, 0.82, 0.83, 0.84, 0.85, 0.86, 0.87, 0.88, 0.89, 0.9, 0.91, 0.92, 0.93, 0.94, 0.95, 0.96, 0.97, 0.98, 0.99, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9 or 10 EU/mg or below can be made.

On the other hand, "substantially inactivated" means that endotoxin toxicity is reduced when it is measured by an optional standard assay, and means that judgement at least that endotoxin is inactivated by 95%, favorably that it is inactivated by 98%, and more favorably that the endotoxin is inactivated by 100% can be made.

A biomaterial or a biomaterial or other product containing collagen as a main ingredient, which makes a collagen-rich organic composition according to the present invention an ingredient material is not in particular restricted as long as it is a biomaterial or a biomaterial or other product containing collagen as a main ingredient, for which the collagen-rich organic composition according to the present invention is made an ingredient material.

Although for directly specifying a collagen-rich organic composition according to the present invention on a basis of structures and/or properties thereof, it is necessary to prepare or create, in multitude, a collagen-rich organic composition according to the present invention and to attempt to specify it on a basis of the structures or properties by using various means, such a test requires even one competent person skilled in the art to carry out the tests many times so that it entails, while taking a lot of time, large economic expenses (See bottom line, page 16 to lines 1-4, page 17, of Chapter 2 Requirements for Claims "2205 Determination on ""Impossible/Impractical Circumstances"" in Examination When a Claim for an Invention of a Product Recites the Manufacturing Process of the Product" Examination Handbook for Patent and Utility Model in Japan, Part II). That is, since for directly specifying a collagen-rich organic composition according to the present invention on a basis of structures or properties thereof, time and expenses incommensurate in light of economy are required of a person skilled in the art on an application being filed, it can be said to be harsh that in circumstances where technology evolves rapidly and where patent acquisition is hard competition on a global scale, an applicant who seeks an application date earlier even by one day under a first-to-file principle is required to carry out such a specifying work (page 10 of Supreme Court (Accepted) 2012 No.1204 or pages 10, 11 of Supreme Court (Accepted) 2012 No.2658, and so on).

Next, a method of producing a collagen-rich organic composition according to the present invention is described. Out of a method of producing a collagen-rich organic composition, according to the present invention, what is configured to be identical or to correspond to the above-mentioned collagen-rich organic composition produced from teeth or bones extracted from a mammal, according to the present invention, as well as to a biomaterial or a biomaterial or other product containing collagen as a main ingredient, which uses the collagen-rich organic composition for an ingredient material has its repeated explanation left out.

A method of producing a collagen-rich organic composition according to the present invention has steps (i)~(iii):
(i) A step of obtaining a pulverization product by pulverizing teeth or bones extracted from a mammal (pulverization product preparation step)
(ii) A step of giving strong alkali treatment to a pulverization product obtained in a pulverization product preparation step (i) (strong alkali treatment step)
(iii) A step of giving demineralization treatment under a negative pressure condition to a pulverization product given strong alkali treatment, obtained in a strong alkali treatment step (ii) (negative pressure demineralization step)

A pulverization product preparation step (i) in a method of producing a collagen-rich organic composition according to the present invention is a step of obtaining a pulverization product by pulverizing teeth or bones extracted from a mammal by using a means publicly-known and conventional in this field, such as a pulverizing machine. In the present invention, the pulverization product is set favorably to 0.05-3mm and more favorably to 0,1-2mm, in particle size.

A strong alkali treatment step (ii) in a method of producing a collagen-rich organic composition according to the present invention is a step of giving agitation treatment in a strongly basic aqueous solution to the pulverization product obtained in the pulverization product preparation step (i). As for the strongly basic aqueous solution, a strongly basic aqueous solution publicly-known and conventional in this field can be used as appropriate, and for such a strongly basic aqueous solution, sodium hydroxide aqueous solution, potassium hydroxide aqueous solution, calcium hydroxide solution, and sodium carbonate aqueous solution can be mentioned, for example.

Additionally, the strong alkali treatment step (ii) may be a step of giving strong alkali treatment under a condition of temperature higher than normal temperature and lower than water boiling point to the pulverization product obtained in the pulverization product preparation step (i). Such a strong alkali treatment step (ii) in the present invention is, specifically, favorably a step of giving strong alkali treatment under a condition of 50~90°C, more favorably a step of giving strong alkali treatment under a condition of 60~80°C, further more favorably a step of giving strong alkali treatment under a condition of 65~75°C, and most favorably a step of giving strong alkali treatment under a condition of 70°C.

A negative pressure demineralization step (iii) in a method of producing a collagen-rich organic composition according to the present invention is a step of giving demineralization treatment under a negative pressure condition to the pulverization product given strong alkali treatment, obtained in the strong alkali treatment step (ii), specifically, a step including a step of giving demineralization treatment while reducing pressure. Since gases (mainly, CO₂) generated during treatment and air having come in can be removed by giving demineralization treatment while reducing pressure, decalcification solution permeates rapidly into a pulverization product, and as a result, an effect of shortening time required for demineralization is obtained. This chemically great effect is that since demineralization can be finished more rapidly without temperature being elevated, it results that substances relatively vulnerable to acids are protected indirectly. The negative pressure demineralization step (iii) in the present invention has only to include a step of giving demineralization treatment while reducing pressure, and for example, a step of giving demineralization treatment under normal pressure and a step of giving demineralization treatment while reducing pressure may be combined with each other. Additionally, the demineralization treatment can be carried out through one or a few steps including a demineralization treatment step publicly-known and conventional in this field, and for example, decalcification solution to be used can, like phosphoric acid, hydrochloric acid, nitric acid, sulfuric acid, formic acid, ethylene diamine tetraacetic acid (EDTA) and besides, ethanol and hydrochloric acid, ethanol and EDTA aqueous solution, be used solely or in combination thereof.

A method of producing a collagen-rich organic composition, according to the present invention may have not only the pulverization product preparation step (i), strong alkali treatment step (ii), and negative pressure demineralization step (iii) above-mentioned but also another step to an extent that features of the present invention are not compromised. As such steps, an additional pulverizing step, a drying step, a pressure-reduction drying step, a cleansing step, a heating step, a cooling step, a neutralizing step, a mixing step, an elution step and so on can be mentioned for example.

Next, a first embodiment of a method of discerning teeth or bones extracted from a mammal to be used as an ingredient material of a biomaterial or a biomaterial or other product containing collagen as a main ingredient mammal, according to the present invention, is described. Out of a method of this first embodiment, what is, as described above, configured to be identical or to correspond to a collagen-rich organic composition produced from teeth or bones extracted from a mammal, and a biomaterial or a biomaterial or other product containing collagen as a main ingredient, which uses the collagen-rich organic composition for an ingredient material, according to the present invention, as well as to a method of producing a collagen-rich organic composition, according to the present invention, has its repeated explanation left out in such a manner of assigning an identical signs.

A method of this first embodiment has steps (iv)~(vii):
(iv) A step of quantifying pyridinoline content (ng/mg) of collagen contained in teeth or bones extracted from a mammal (pyridinoline content quantification step)
(v) A step of quantifying pentosidine content (ng/mg) of the collagen contained in teeth or bones extracted from a mammal (pentosidine content quantification step)
(vi) A step of calculating, from a PYD being a value of pyridinoline content (ng/mg) quantified in a pyridinoline content quantification step (iv) and a PEN being a value of pentosidine content (ng/mg) quantified in a pentosidine content quantification step (v), a PpP (pyridinoline content (ng/mg)/ pentosidine content (ng/mg)) being a weight ratio (weight ratio calculation step)
(vii) a step of selecting, when a PYD being a value of pyridinoline content (ng/mg) quantified in a pyridinoline content quantification step (iv) or a PYD being a value of pyridinoline content (ng/mg) quantified in a pyridinoline content quantification step (iv) and a PpP (pyridinoline content (ng/mg)/ pentosidine content (ng/mg)) being a value of a weight ratio calculated in a weight ratio calculation step (vi) are specified values, the teeth or bones extracted from a mammal for an ingredient material of a biomaterial or a biomaterial or other product containing collagen as a main ingredient (teeth/bones selection step)

In a pyridinoline content quantification step (iv) and a pentosidine content quantification step (v) in a method of this first embodiment, a quantification method of pyridinoline content (ng/mg) of collagen contained in teeth or bones extracted from a mammal and that of pentosidine content (ng/mg) thereof are not restricted in particular as long as those quantifications are allowed, and as such a quantification method, a high-performance liquid chromatography with a fluorescence detector (HPLC-Flu) using heptafluorobutyric acid and formic acid as an ion-pairing reagent can be, for example, adopted.

In a weight ratio calculation step (vi) in a method of this first embodiment, a PYD being a quantification value of pyridinoline content (ng/mg), quantified in a pyridinoline content quantification step (iv), of collagen contained in teeth or bones extracted from a mammal is divided by a PEN being a quantification value of pentosidine content (ng/mg), quantified in a pentosidine content quantification step (v), of collagen contained in teeth or bones extracted from a mammal, by which a PpP (pyridinoline content (ng/mg)/ pentosidine content (ng/mg)) being a value of a weight ratio between a quantification value of pyridinoline content (ng/mg) and a quantification value of pentosidine content (ng/mg) is calculated.

In a teeth/bones selection step (vii) in a method of this first embodiment, the teeth or bones extracted from a mammal are selected for an ingredient material of a biomaterial or a biomaterial or other product containing collagen as a main ingredient, when a PYD being a value of pyridinoline content (ng/mg) quantified in a pyridinoline content quantification step (iv) or a PYD being a value of pyridinoline content (ng/mg) quantified in a pyridinoline content quantification step (iv) and a PpP (pyridinoline content (ng/mg)/ pentosidine content (ng/mg)) being a value of a weight ratio calculated in a weight ratio calculation step (vi).

"Specified values" mentioned in the teeth/bones selection step (vii) are defined in a quantification method of pyridinoline content (ng/mg) of collagen contained in teeth or bones extracted from a mammal and that of pentosidine content (ng/mg) thereof, and as for the specifies values, PYD>100 or PYD>100 and PpP>740, respectively, for example, when a pyridinoline content quantification step (iv) and a pentosidine content quantification step (v) are a step of carrying out quantification with a high-performance liquid chromatography with a fluorescence detector (HPLC-Flu) using heptafluorobutyric acid and formic acid as an ion-pairing reagent.

A method of this first embodiment may, like a method of producing a collagen-rich organic composition, according to the present invention, have not only the pyridinoline content quantification step (iv), pentosidine content quantification step (v), weight ratio calculation step (vi), and teeth/bones selection step (vii) above-mentioned but also another step to an extent that features of the present invention are not compromised.

Next, a second embodiment of a method of discerning teeth or bones extracted from a mammal to be used as an ingredient material of a biomaterial or a biomaterial or other product containing collagen as a main ingredient mammal is described. This second embodiment has steps (i)~(vii):
(i) A step of obtaining a pulverization product by pulverizing teeth or bones extracted from a mammal (pulverization product preparation step)
(ii) A step of giving strong alkali treatment to a pulverization product obtained in a pulverization product preparation step (i) (strong alkali treatment step)
(iii) A step of obtaining a collagen-rich organic composition by giving demineralization treatment under a negative pressure condition to a pulverization product given strong alkali treatment, obtained in a strong alkali treatment step (ii) (negative pressure demineralization step)
(iv) A step of quantifying pyridinoline content (ng/mg) of collagen contained in a collagen-rich organic composition obtained in a negative pressure demineralization step (iii) (pyridinoline content quantification step)
(v) A step of quantifying pentosidine content (ng/mg) of collagen contained in a collagen-rich organic composition obtained in a negative pressure demineralization step (iii) (pentosidine content quantification step)
(vi) A step of calculating, from a PYD being a value of pyridinoline content (ng/mg) quantified in a pyridinoline content quantification step (iv) and a PEN being a value of pentosidine content (ng/mg) quantified in a pentosidine content quantification step (v), a PpP (pyridinoline content (ng/mg)/ pentosidine content (ng/mg)) being a weight ratio (weight ratio calculation step)
(vii) a step of selecting, when a PYD being a value of pyridinoline content (ng/mg) quantified in a pyridinoline content quantification step (iv) or a PYD being a value of pyridinoline content (ng/mg) quantified in a pyridinoline content quantification step (iv) and a PpP (pyridinoline content (ng/mg)/ pentosidine content (ng/mg)) being a value of a weight ratio calculated in a weight ratio calculation step (vi) are specified values, the teeth or bones extracted from a mammal for an ingredient material of a biomaterial or a biomaterial or other product containing collagen as a main ingredient (teeth/bones selection step)

Out of a method of this second embodiment, what is, as described above, configured to be identical or to correspond to a collagen-rich organic composition produced from teeth or bones extracted from a mammal, and a biomaterial or a biomaterial or other product containing collagen as a main ingredient, which uses the collagen-rich organic composition for an ingredient material, according to the present invention, as well as to a method of producing a collagen-rich organic composition, and a method of discerning teeth or bones extracted from a mammal to be used as an ingredient material of a biomaterial or a biomaterial or other product containing collagen as a main ingredient mammal, according to the present invention, has its repeated explanation left out in such a manner of assigning an identical signs.

A pulverization product preparation step (i), a strong alkali treatment step (ii), and a negative pressure demineralization step (iii), which are a configuration requirement of a method of this second embodiment, correspond to a pulverization product preparation step (i), a strong alkali treatment step (ii), and a negative pressure demineralization step (iii), which are a configuration requirement of a method of producing a collagen-rich organic composition, according to the present invention, and a pyridinoline content quantification step (iv), a pentosidine content quantification step (v), a weight ratio calculation step (vi), and a teeth/bones selection step (vii), which are a configuration requirement of a method of this second embodiment, correspond to a pyridinoline content quantification step (iv), a pentosidine content quantification step (v), a weight ratio calculation step (vi), and a teeth/bones selection step (vii), which are a configuration requirement of a first embodiment of a method of discerning teeth or bones extracted from a mammal to be used as an ingredient material of a biomaterial or a biomaterial or other product containing collagen as a main ingredient mammal, according to the present invention.

A method of this second embodiment may also, like a first embodiment of a method of producing a collagen-rich organic composition, according to the present invention and a method of discerning teeth or bones extracted from a mammal to be used as an ingredient material of a biomaterial or a biomaterial or other product containing collagen as a main ingredient mammal, according to the present invention, have not only the pulverization product preparation step (i), strong alkali treatment step (ii), negative pressure demineralization step (iii), pyridinoline content quantification step (iv), pentosidine content quantification step (v), weight ratio calculation step (vi), and teeth/bones selection step (vii) above-mentioned but also another step to an extent that features of the present invention are not compromised.

Next, a method of using, as an indicator for assessing a potential morbidity risk of a disease in a mammal and/or whether a mammal is potentially in good health or not, a quantification value of pyridinoline content of collagen contained in teeth of a mammal, or a quantification value of pyridinoline content of collagen contained in teeth of a mammal as well as a weight ratio (pyridinoline content/ pentosidine content) calculated from a quantification value of pyridinoline content of collagen contained in teeth of a mammal and a quantification value of pentosidine content thereof, according to the present invention has steps (viii)~(xiv):
(viii) A step of obtaining, by quantifying pyridinoline content (ng/mg) of collagen contained in teeth of a mammal to be assessed for a potential morbidity risk of a disease and/or whether a mammal is potentially in good health or not, a PYD being a quantification value (assessment subject pyridinoline content quantification step)
(ix) A step of obtaining, by quantifying pentosidine content (ng/mg) of the collagen contained in teeth of a mammal to be assessed, a PEN being a quantification value (assessment subject pentosidine content quantification step)
(x) A step of calculating, from a PYD obtained in an assessment subject pyridinoline content quantification step (viii) and a PEN obtained in an assessment subject pentosidine content quantification step (ix), a PpP (pyridinoline content (ng/mg)/ pentosidine content (ng/mg)) being a value of a weight ratio (assessment subject weight ratio calculation step)
(xi) A step of obtaining, by quantifying pyridinoline content (ng/mg) of collagen contained in teeth of 1 or 2 or more mammals selected from a mammal not affected by a disease, a mammal affected by a disease, a mammal in good health, and a mammal not in good health, a R-PYD being a reference quantification value (reference pyridinoline content quantification step)
(xii) A step of obtaining, by quantifying pentosidine content (ng/mg) of collagen contained in teeth of 1 or 2 or more mammals selected from a mammal not affected by a disease, a mammal affected by a disease, a mammal in good health, and a mammal not in good health, a R-PEN being a reference quantification value (reference pentosidine content quantification step)
(xiii) A step of calculating, from a R-PYD obtained in a reference pyridinoline content quantification step (xi) and a R-PEN obtained in a reference pentosidine content quantification step (xii), a R-PpP (pyridinoline content (ng/mg)/ pentosidine content (ng/mg)) being a reference value of a weight ratio (reference weight ratio calculation step)
(xiv) A step of comparing a PYD obtained in an assessment subject pyridinoline content quantification step (viii) and a R-PYD obtained in a reference pyridinoline content quantification step (xi), or comparing a PYD obtained in an assessment subject pyridinoline content quantification step (viii) and a R-PYD obtained in a reference pyridinoline content quantification step (xi) as well as a PpP calculated in an assessment subject weight ratio calculation step (x) and a R-PpP calculated in a reference weight ratio calculation step (xiii) (PYD/weight ratio comparison step)

Out of a method of using, as an indicator for assessing a potential morbidity risk of a disease in a mammal and/or whether a mammal is potentially in good health or not, a quantification value of pyridinoline content of collagen contained in teeth of a mammal, or a quantification value of pyridinoline content of collagen contained in teeth of a mammal as well as a weight ratio (pyridinoline content/ pentosidine content) calculated from a quantification value of pyridinoline content of collagen contained in teeth of a mammal and a quantification value of pentosidine content thereof, what is, as described above, configured to be identical or to correspond to a first embodiment and second embodiment of a collagen-rich organic composition produced from teeth or bones extracted from a mammal, and a biomaterial or a biomaterial or other product containing collagen as a main ingredient, which uses the collagen-rich organic composition for an ingredient material, according to the present invention, as well as to a method of producing a collagen-rich organic composition, and a method of discerning teeth or bones extracted from a mammal to be used as an ingredient material of a biomaterial or a biomaterial or other product containing collagen as a main ingredient mammal, according to the present invention, has its repeated explanation left out in such a manner of assigning an identical signs.

In the present invention, a "potential morbidity risk" does not refer to a state of being affected by a disease, but means a state of having a risk such as a host narrowly not leading to diseases due to his/her defense response acting maximally thereagainst, and signifies a state of being almost unable, even in subjective findings, objective findings, and conventional general examination findings, to ascertain being affected by a disease.

Additionally, in the present invention, being "potentially in good health" means such a state as not reaching a state of being not in good health, and signifies a state of being almost unable, even in subjective findings, objective findings, and conventional general examination findings, to ascertain being in poor health, whereas in the present invention, being "potentially not in good health" means such a state as not reaching a state of being in good health, and signifies a state of being almost unable, even in subjective findings, objective findings, and conventional general examination findings, to ascertain being in good health.

An assessment subject pyridinoline content quantification step (viii) in a method of using, as an indicator for assessing a potential morbidity risk of a disease in a mammal and/or whether a mammal is potentially in good health or not, a quantification value of pyridinoline content of collagen contained in teeth of a mammal, or a quantification value of pyridinoline content of collagen contained in teeth of a mammal as well as a weight ratio (pyridinoline content/ pentosidine content) calculated from a quantification value of pyridinoline content of collagen contained in teeth of a mammal and a quantification value of pentosidine content thereof, according to the present invention is a step corresponding to a pyridinoline content quantification step (iv) of a first embodiment and second embodiment of a method of discerning teeth or bones extracted from a mammal to be used as an ingredient material of a biomaterial or a biomaterial or other product containing collagen as a main ingredient mammal, according to the present invention.

Additionally, an assessment subject pentosidine content quantification step (ix) in a method of using, as an indicator for assessing a potential morbidity risk of a disease in a mammal and/or whether a mammal is potentially in good health or not, a quantification value of pyridinoline content of collagen contained in teeth of a mammal, or a quantification value of pyridinoline content of collagen contained in teeth of a mammal as well as a weight ratio (pyridinoline content/ pentosidine content) calculated from a quantification value of pyridinoline content of collagen contained in teeth of a mammal and a quantification value of pentosidine content thereof, according to the present invention is a step corresponding to a pentosidine content quantification step (v) of a first embodiment and second embodiment of a method of discerning teeth or bones extracted from a mammal to be used as an ingredient material of a biomaterial or a biomaterial or other product containing collagen as a main ingredient mammal, according to the present invention.

Additionally, an assessment subject weight ratio calculation step (x) in a method of using, as an indicator for assessing a potential morbidity risk of a disease in a mammal and/or whether a mammal is potentially in good health or not, a quantification value of pyridinoline content of collagen contained in teeth of a mammal, or a quantification value of pyridinoline content of collagen contained in teeth of a mammal as well as a weight ratio (pyridinoline content/ pentosidine content) calculated from a quantification value of pyridinoline content of collagen contained in teeth of a mammal and a quantification value of pentosidine content thereof, according to the present invention is a step corresponding to a weight ratio calculation step (vi) of a first embodiment and second embodiment of a method of discerning teeth or bones extracted from a mammal to be used as an ingredient material of a biomaterial or a biomaterial or other product containing collagen as a main ingredient mammal, according to the present invention.

Additionally, a reference pyridinoline content quantification step (xi) in a method of using, as an indicator for assessing a potential morbidity risk of a disease in a mammal and/or whether a mammal is potentially in good health or not, a quantification value of pyridinoline content of collagen contained in teeth of a mammal, or a quantification value of pyridinoline content of collagen contained in teeth of a mammal as well as a weight ratio (pyridinoline content/ pentosidine content) calculated from a quantification value of pyridinoline content of collagen contained in teeth of a mammal and a quantification value of pentosidine content thereof, according to the present invention corresponds to a pyridinoline content quantification step (iv) of a first embodiment and second embodiment of a method of discerning teeth or bones extracted from a mammal to be used as an ingredient material of a biomaterial or a biomaterial or other product containing collagen as a main ingredient mammal, according to the present invention, as well as an assessment subject pyridinoline content quantification step (viii) in a method of using, as an indicator for assessing a potential morbidity risk of a disease in a mammal and/or whether a mammal is potentially in good health or not, a quantification value of pyridinoline content of collagen contained in teeth of a mammal, or a quantification value of pyridinoline content of collagen contained in teeth of a mammal as well as a weight ratio (pyridinoline content/ pentosidine content) calculated from a quantification value of pyridinoline content of collagen contained in teeth of a mammal and a quantification value of pentosidine content thereof, according to the present invention.

Additionally, a reference pentosidine content quantification step (xii) in a method of using, as an indicator for assessing a potential morbidity risk of a disease in a mammal and/or whether a mammal is potentially in good health or not, a quantification value of pyridinoline content of collagen contained in teeth of a mammal, or a quantification value of pyridinoline content of collagen contained in teeth of a mammal as well as a weight ratio (pyridinoline content/ pentosidine content) calculated from a quantification value of pyridinoline content of collagen contained in teeth of a mammal and a quantification value of pentosidine content thereof, according to the present invention corresponds to a pentosidine content quantification step (v) of a first embodiment and second embodiment of a method of discerning teeth or bones extracted from a mammal to be used as an ingredient material of a biomaterial or a biomaterial or other product containing collagen as a main ingredient mammal, according to the present invention, as well as an assessment subject pentosidine content quantification step (ix) in a method of using, as an indicator for assessing a potential morbidity risk of a disease in a mammal and/or whether a mammal is potentially in good health or not, a quantification value of pyridinoline content of collagen contained in teeth of a mammal, or a quantification value of pyridinoline content of collagen contained in teeth of a mammal as well as a weight ratio (pyridinoline content/ pentosidine content) calculated from a quantification value of pyridinoline content of collagen contained in teeth of a mammal and a quantification value of pentosidine content thereof, according to the present invention.

"Collagen contained in teeth of 1 or 2 or more mammals selected from a mammal not affected by a disease, a mammal affected by a disease, a mammal in good health, and a mammal not in good health" in a reference pentosidine content quantification step (xii) may be "collagen contained in teeth of 1 or 2 or more mammals selected from a mammal not affected by a disease, a mammal affected by a disease, a mammal in good health, and a mammal not in good health" different from that in a reference pyridinoline content quantification step (xi), and may be "collagen contained in teeth of 1 or 2 or more mammals selected from a mammal not affected by a disease, a mammal affected by a disease, a mammal in good health, and a mammal not in good health" identical thereto.

Additionally, in any step, a "mammal not affected by a disease" includes a "self being a mammal not affected by a disease," a "mammal affected by a disease" includes a "self being a mammal affected by a disease," a "mammal in good health" includes a "self being a mammal in good health," and a "mammal not in good health" includes a "self being a mammal not in good health."

Additionally, a reference weight ratio calculation step (xiii) in a method of using, as an indicator for assessing a potential morbidity risk of a disease in a mammal and/or whether a mammal is potentially in good health or not, a quantification value of pyridinoline content of collagen contained in teeth of a mammal, or a quantification value of pyridinoline content of collagen contained in teeth of a mammal as well as a weight ratio (pyridinoline content/ pentosidine content) calculated from a quantification value of pyridinoline content of collagen contained in teeth of a mammal and a quantification value of pentosidine content thereof, according to the present invention corresponds to a weight ratio calculation step (vi) of a first embodiment and second embodiment of a method of discerning teeth or bones extracted from a mammal to be used as an ingredient material of a biomaterial or a biomaterial or other product containing collagen as a main ingredient mammal, according to the present invention, as well as to an assessment subject weight ratio calculation step (x) in a method of using, as an indicator for assessing a potential morbidity risk of a disease in a mammal and/or whether a mammal is potentially in good health or not, a quantification value of pyridinoline content of collagen contained in teeth of a mammal, or a quantification value of pyridinoline content of collagen contained in teeth of a mammal as well as a weight ratio (pyridinoline content/ pentosidine content) calculated from a quantification value of pyridinoline content of collagen contained in teeth of a mammal and a quantification value of pentosidine content thereof, according to the present invention.

Additionally, a PYD/weight ratio comparison step (xiv) in a method of using, as an indicator for assessing a potential morbidity risk of a disease in a mammal and/or whether a mammal is potentially in good health or not, a quantification value of pyridinoline content of collagen contained in teeth of a mammal, or a quantification value of pyridinoline content of collagen contained in teeth of a mammal as well as a weight ratio (pyridinoline content/ pentosidine content) calculated from a quantification value of pyridinoline content of collagen contained in teeth of a mammal and a quantification value of pentosidine content thereof, according to the present invention is a step of comparing a PpP (pyridinoline content (ng/mg)/ pentosidine content (ng/mg)) being a value of a weight ratio calculated in an assessment subject weight ratio calculation step (x) and a R-PpP (pyridinoline content (ng/mg)/ pentosidine content (ng/mg)) being a reference value of a weight ratio calculated in a reference weight ratio calculation step (xiii).

An assessment subject pyridinoline content quantification step (viii), an assessment subject pentosidine content quantification step (ix), a reference pyridinoline content quantification step (xi), and a reference pentosidine content quantification step (xii) in a method of using, as an indicator for assessing a potential morbidity risk of a disease in a mammal and/or whether a mammal is potentially in good health or not, a quantification value of pyridinoline content of collagen contained in teeth of a mammal, or a quantification value of pyridinoline content of collagen contained in teeth of a mammal as well as a weight ratio (pyridinoline content/ pentosidine content) calculated from a quantification value of pyridinoline content of collagen contained in teeth of a mammal and a quantification value of pentosidine content thereof, according to the present invention can be carried out on teeth extracted from a mammal to be assessed for a potential morbidity risk of a disease, a mammal not affected by a disease, a mammal affected by a disease, a mammal in good health, and a mammal not in good health, and besides, can be carried out even in modes such as cutting, contact, and non-contact, for example.

A method of using, as an indicator for assessing a potential morbidity risk of a disease in a mammal and/or whether a mammal is potentially in good health or not, a quantification value of pyridinoline content of collagen contained in teeth of a mammal, or a quantification value of pyridinoline content of collagen contained in teeth of a mammal as well as a weight ratio (pyridinoline content/ pentosidine content) calculated from a quantification value of pyridinoline content of collagen contained in teeth of a mammal and a quantification value of pentosidine content thereof, according to the present invention may also, like a first embodiment and second embodiment of a method of producing a collagen-rich organic composition, according to the present invention and a method of discerning teeth or bones extracted from a mammal to be used as an ingredient material of a biomaterial or a biomaterial or other product containing collagen as a main ingredientmammal, according to the present invention, have not only the assessment subject pyridinoline content quantification step (viii), assessment subject pentosidine content quantification step (ix), assessment subject weight ratio calculation step (x), reference pyridinoline content quantification step (xi), reference pentosidine content quantification step (xii), reference weight ratio calculation step (xiii), and PYD/weight ratio comparison step (xiv) above-mentioned but also another step to an extent that features of the present invention are not compromised.

Why a method of using, as an indicator for assessing a potential morbidity risk of a disease in a mammal and/or whether a mammal is potentially in good health or not, a quantification value of pyridinoline content of collagen contained in teeth of a mammal, or a quantification value of pyridinoline content of collagen contained in teeth of a mammal as well as a weight ratio (pyridinoline content/ pentosidine content) calculated from a quantification value of pyridinoline content of collagen contained in teeth of a mammal and a quantification value of pentosidine content thereof, according to the present invention is restricted to "teeth" is that both pyridinoline crosslinking and pentosidine crosslinking in type I collagen are a crosslinking reaction accelerated by factors such as internal body heat due to body temperature and sugars supplied in a transvascular manner, and are a crosslinking reaction caused not locally but homogeneously in a whole body, above all, that it is collagen contained in teeth (especially, dentin) that is believed to reflect most accurately accumulation of pyridinoline crosslinking molecules and pentosidine crosslinking molecules. That is because pyridinoline crosslinking molecules and pentosidine crosslinking molecules are accumulated in collagen contained in teeth (especially, dentin) since teeth are sole tissues not being incorporated in a metabolic cycle in a living organism and are neither absorbed nor decomposed once formed.

Further, collagen of teeth (especially, dentin) is insoluble as compared with that of bones. Pepsin digestion under a condition of 0.01N hydrochloric acid, 4°C, 72 hours of treatment time solubilizes approximately 35% of collagen as for adult bovines' bones, but only solubilizes 5.6% of collagen as for dentin of adult bovines' teeth. Additionally, insoluble collagen of skins, Achilles tendons, and so on is swollen 4~8 times in volume under a condition of pH2, and insoluble collagen of adult bovines' bones is swollen 1.2 times in volume thereunder, whereas insoluble collagen of adult bovines' dentin is not swollen at all thereunder (Yutaka Nagai and Daizaburo Fujimoto (eds.), Collagen Experimental Methods, Kodansha Scientific, pp. 21-22). Additionally, dentin collagen of teeth is highest in content rate of pyridinoline crosslinking molecules in living organisms. Accordingly, it is with reason to assess a potential morbidity risk of a disease in a mammal and/or whether a mammal is potentially in good health or not, by using, as an indicator, a PYD being a quantification value of pyridinoline content of collagen contained in teeth of a mammal, or a PYD being a quantification value of pyridinoline content of collagen contained in teeth of a mammal as well as a PpP (pyridinoline content/ pentosidine content) being a value of a weight ratio calculated from a PYD being a quantification value of pyridinoline content of collagen contained in teeth of a mammal and a PEN being a quantification value of pentosidine content thereof.

On the other hand, reasons for excluding bones are described. Although numerical values of type I collagen C-terminal telopeptides (ICTP) in blood (serum) and NTx (type I collagen crosslinking N-telopeptide) in urine are widely utilized as an indicator of osteoporosis, these are substances to be released into blood/urine as collagen decomposition products made by matrix metalloproteases (MMPs) and are substances containing pyridinoline crosslinking molecules. In addition to a fact that this makes it understandable that pyridinoline crosslinking molecules and pentosidine crosslinking molecules contained in bone content collagen are partially cleared through metabolism, collagen high in purity is, when extracted bones are used, made to be much harder to refine since bones are organs containing a large quantity of lipid originating from yellow bone marrows so that they are not easy in defatting action.

When collagen contained in teeth of a mammal is large in pyridinoline content and small in pentosidine content, the mammal has its homeostasis enhanced as an individual itself and has collagen where chronic inflammation is not easily induced, and can be assessed to be low in a potential risk of coming down with various diseases and can be also determined to be potentially in good health. On another side, when collagen contained in teeth of a mammal is small in pyridinoline content and large in pentosidine content, the mammal has its homeostasis lowered as an individual itself and has collagen where chronic inflammation is easily induced, and can be assessed to be high in a potential risk of coming down with various diseases and can be also determined to be potentially not in good health, and therefore, as an indicator for assessing a potential morbidity risk of a disease in a mammal and/or whether a mammal is potentially in good health or not, a PYD being a quantification value of pyridinoline content of collagen contained in teeth of a mammal, or a PYD being a quantification value of pyridinoline content of collagen contained in teeth of a mammal as well as a PpP (pyridinoline content/ pentosidine content) being a value of a weight ratio calculated from a PYD being a quantification value of pyridinoline content of collagen contained in teeth of a mammal and a PEN being a quantification value of pentosidine content thereof can be used. A method of using, as an indicator for assessing a potential morbidity risk of a disease in a mammal according to the present invention, a PYD being a quantification value of pyridinoline content of collagen contained in teeth of a mammal, or a PYD being a quantification value of pyridinoline content of collagen contained in teeth of a mammal as well as a PpP (pyridinoline content/ pentosidine content) being a value of a weight ratio calculated from a PYD being a quantification value of pyridinoline content of collagen contained in teeth of a mammal and a PEN being a quantification value of pentosidine content thereof, and a method of using, as an indicator for assessing whether a mammal is potentially in good health or not according to the present invention, a PYD being a quantification value of pyridinoline content of collagen contained in teeth of a mammal, or a PYD being a quantification value of pyridinoline content of collagen contained in teeth of a mammal as well as a PpP (pyridinoline content/ pentosidine content) being a value of a weight ratio calculated from a PYD being a quantification value of pyridinoline content of collagen contained in teeth of a mammal and a PEN being a quantification value of pentosidine content thereof, may be independent from and may be related to each other.

A collagen-rich organic composition produced from teeth or bones extracted from a mammal, a biomaterial or a biomaterial or other product containing collagen as a main ingredient, which uses the collagen-rich organic composition for an ingredient material, according to the present invention, a method of producing a collagen-rich organic composition, a method of discerning teeth or bones extracted from a mammal to be used as an ingredient material of a biomaterial or a biomaterial or other product containing collagen as a main ingredient mammal, according to the present invention, and a method of using, as an indicator for assessing a potential morbidity risk of a disease in a mammal and/or whether a mammal is potentially in good health or not, a quantification value of pyridinoline content of collagen contained in teeth of a mammal, or a quantification value of pyridinoline content of collagen contained in teeth of a mammal as well as a weight ratio (pyridinoline content/ pentosidine content) calculated from a quantification value of pyridinoline content of collagen contained in teeth of a mammal and a quantification value of pentosidine content thereof, according to the present invention are described below on a basis of an embodiment example. Technical scope of the present invention is not restricted by embodiments shown by these embodiment examples.

### [Embodiment Example]

### <Embodiment Example 1> Measurement of quantity of pyridinoline and pentosidine in collagen derived from of teeth (mainly, dentin) of humans, bovines, and swine

By following steps below, measurement was carried out on quantity of pyridinoline and pentosidine in collagen (n=62) derived from teeth (mainly, dentin) of 18 cases of humans, 36 cases of bovines (therein, 19 cases of bovine SRM (Hayakita Factory, Hokkaido Livestock Corporation), 17 cases of TOKACHIWAKAUSHI (a male Holstein with a Japan-registered trademark and through early fattening (14 months old); JA Tokachi Shimizu)), and 8 cases of livestock swine (six months old; Hayakita Factory, Hokkaido Livestock Corporation), soft tissue-derived Teruplug (n=3, bovine dermis-derived atelocollagen, telopeptide not-containing type treatment; Olympus Terumo Biomaterials Corp.), high-grade gelatin (n=3, porcine-skin acid treatment, telopeptide preservation type treatment method, low-endotoxin collagen; Nippi, Incorporated), TOKACHIWAKAWUSHI (a Japan-registered trademark)'s gums (n=3; JA Tokachi Shimizu), and TOKACHIWAKAWUSHI (a Japan-registered trademark)'s muscles (n=3; JA Tokachi Shimizu).

### 1. Preparation of collagen powder

[1-1] By using a pulverizing machine, a hard tissue was pulverized to a degree of 1 mm in particle size.
[1-2] In a 1% sodium carbonate aqueous solution, stirring treatment was carried out for several hours under a condition of 70°C and soft tissues attached were removed.
[1-3] Demineralization treatment was carried out. To go into details, the demineralization treatment was, by using decalcification solution containing 1M phosphoric acid and ethanol, carried out for several hours while depressurization was being carried out, and thereafter, the demineralization treatment was, by substituting the decalcification solution diluted several times, carried out for several hours while depressurization was being carried out. Followingly, demineralization was carried out completely by giving a shake for about one day and night under a room-temperature condition with the above-mentioned decalcification solution diluted five times. Then, a wash was, by using 5% ethanol, made several times under a room-temperature condition.
[1-4] The hard tissue demineralized was moved into a mortar, rubbed and crushed in 70% ethanol, and thereby powdered finely.
[1-5] It was stirred in 100% ethanol and washed, as well as defatted and dehydrated.
[1-6] Ethanol left in collagen was completely removed by being dried, and thereby, dried collagen powder was obtained.

### 2. Preparation of an analytical sample

[2-1] The dried collagen powder obtained in [1-6] was hydrolyzed. To go into details, the dried collagen powder obtained was measured and taken into a test tube, was fully swollen by means of a small amount of 6N hydrochloric acid under deaeration, and thereafter, a suitable amount of 6N hydrochloric acid was added to the dried collagen and the test tube was being welded under deaeration to be sealed. After the test tube sealed was given overnight heating treatment under a condition of 110°C one day and one night to carry out hydrolysis, the test tube was cooled to room temperature and severed, and surplus hydrochloric acid was removed by being dried under reduced pressure while being warmed so that a collagen hydrolysate was obtained.
[2-2] Collagen hydrolysate obtained was solubilized in 10% methanol aqueous solution, and centrifugation was made for 5 minutes at 12000 rpm under room temperature to make a supernatant obtained a sample.
[2-3] A known amount of a standard substance was added to the sample, and thereby, an extraction rate and a calibration curve were obtained to modify collagen contents as appropriate.

### 3. Quantification of pyridinoline content and pentosidine content through a high-performance liquid chromatography with a fluorescence detector (HPLC-Flu) using heptafluorobutyric acid and formic acid as an ion-pairing reagent, and calculation of weight ratio therebetween

A ultrapure water (mobile phase A) containing 0.05% heptafluorobutyric acid, and a methanol/ethanol 1:1 mixture solution (mobile phase B) containing 0.05% formic acid were used as a mobile phase, and thereby, liquid delivery was carried out in a gradient setting to make detection. Specifically, the liquid delivery was initiated with a detection column temperature being 40°C and a flow rate being 0.5 mL/min and in a proportion of mobile phase A 90% and mobile phase B 10%, and went on for 0.5 minutes. Then, after the mobile phase B was increased to 22% by spending 1 minute and a concentration gradient was applied thereby, the liquid delivery was carried out for 7.5 minutes as it was. Next, the mobile phase B was increased to 80% by spending 1 minute, and further, the liquid delivery was carried out for 1 minute.

Finally, after the liquid delivery was carried out in a proportion of mobile phase A 10% and mobile phase B 90%, it had subsequently its mobile phase B decreased to 10% and was equilibrated for 4 minutes in a proportion of mobile phase A 90% and mobile phase B 10%.

Followingly, detection was carried out for 15 minutes in total for one sample. Cadenza CD-C18 with its length 150mm and its inner diameter 3mm was used for a separation column and a fluorescent detector was used for detection. Monitoring was carried out for first-half 8 minutes at 295nm in excitation wavelength and 395nm in emission wavelength, and for second-half 7 minutes at 325nm in excitation wavelength and 385nm in emission wavelength.

The monitoring made contained pyridinoline be detected at a retention time of about 6.5 minutes or around and contained pentosidine be detected at a retention time of about 9.1 minutes or around. From a pyridinoline content and pentosidine content obtained, a weight ratio therebetween (pyridinoline content(ng/mg)/ pentosidine content(ng/mg)) was obtained.

Although permanent teeth are made to be a measurement target with regard to human teeth, it can be thought in consideration of a formation period of permanent tooth dentin that dentin formation is initiated in a tooth germ inside a jawbone at an age around 10 years old on average and along therewith glycation crosslinking formation is also initiated (Chronology of Deciduous and Permanent Dentition in Japanese Children: II, The Japanese Journal of Pediatric Dentistry 57(3), 363-373, 2019, 363). On the other hand, since a similar matter can be said 18 months or more after birth on average with regard to lower jaw teeth of bovine SRM as well, Table 1 shown below was used as a formula on an occasion of converting a yearly age into a monthly age with regard to specified teeth of humans and bovine SRM.

**[Table 1]**

| **Teeth monthly age conversion formula (Real yearly age x 12 - Tooth type number corresponding to Table below)** | | | |
|---|---|---|---|
| | Tooth type | Male | Female |
| Human upper jaw | 1 | 86 | 83 |
| | 2 | 100 | 95 |
| | 3 | 132 | 123 |
| | 4 | 124 | 120 |
| | 5 | 141 | 138 |
| | 6 | 87 | 85 |
| | 7 | 159 | 156 |
| | 8 | 204 | 204 |
| Human lower jaw | 1 | 75 | 72 |
| | 2 | 87 | 84 |
| | 3 | 123 | 114 |
| | 4 | 125 | 121 |
| | 5 | 140 | 140 |
| | 6 | 80 | 75 |
| | 7 | 151 | 150 |
| | 8 | 204 | 204 |
| Bovine SRM | Lower 1 | 18 | 18 |
| TOKACHIWAKAUSHI | Deciduous teeth | Conversion NOT Required | Conversion NOT Required |
| Livestock swine | Deciduous teeth | Conversion NOT Required | Conversion NOT Required |

A PYD being a quantification value of pyridinoline content (ng/mg), and a PEN being a quantification value of pentosidine content (ng/mg), which were quantified with a high-performance liquid chromatography with a fluorescence detector (HPLC-Flu) using heptafluorobutyric acid and formic acid as an ion-pairing reagent and were contained in a collagen-rich organic composition derived from dentin of teeth of 18 cases of humans, as well as a PpP (pyridinoline content (ng/mg)/ pentosidine content (ng/mg)) being a value of a weight ratio calculated from a PYD being a quantification value of pyridinoline content (ng/mg) and a PEN being a quantification value of pentosidine content (ng/mg), are shown in Table 2 shown below.

**[Table 2]**

| Monthly age | 24 | 96 | 175 | 175 | 176 | 176 | 180 | 192 | 300 | 324 |
|---|---|---|---|---|---|---|---|---|---|---|
| PYD | 391.73948 | 409.22903 | 42351531 | 44253674 | 3387585 | 432.10558 | 441.49541 | 463.81418 | 408.00929 | 455.65303 |
| PEN | 03136904 | 0.4107021 | 0.5122305 | 0.5982188 | 03196336 | 0.4801356 | 06203436 | 0.6068596 | 0.6810585 | 0.8186816 |
| PpP | 1248.8094 | 996.41329 | 826.80605 | 739.75737 | 1059.8339 | 899.96576 | 711.695 | 764.28585 | 599.08113 | 556.56927 |

| Monthly age | 336 | 456 | 516 | 636 | 844 | 864 | 876 | 876 |
|---|---|---|---|---|---|---|---|---|
| PYD | 446.70943 | 376.20819 | 381.36663 | 326.06822 | 455.37939 | 386.13481 | 312.4233 | 313.4794 |
| PEN | 130.85563 | 1.2213554 | 1.0250463 | 0.8652362 | 0.709146 | 1.3836648 | 0.9327651 | 1.7724193 |
| PpP | 341.37577 | 308.02516 | 372.04818 | 376.85457 | 64215184 | 279.06672 | 33494318 | 176.86526 |

A PYD being a quantification value of pyridinoline content (ng/mg), and a PEN being a quantification value of pentosidine content (ng/mg), which were quantified with a high-performance liquid chromatography with a fluorescence detector (HPLC-Flu) using heptafluorobutyric acid and formic acid as an ion-pairing reagent and were contained in a collagen-rich organic composition derived from dentin of teeth of 19 cases of bovine SRM, as well as a PpP (pyridinoline content (ng/mg)/ pentosidine content (ng/mg)) being a value of a weight ratio calculated from a PYD being a quantification value of pyridinoline content (ng/mg) and a PEN being a quantification value of pentosidine content (ng/mg), are shown in Table 3 shown below.

**[Table 3]**

| Monthly age | 2425 | 30.28 | 32.23 | 33.08 | 33.14 | 35.07 | 44.03 | 47.11 | 47.14 | 52.12 |
|---|---|---|---|---|---|---|---|---|---|---|
| PYD | 188.27679 | 283.7318 | 215.25452 | 2792532 | 213.59049 | 27082968 | 270.20892 | 225.84465 | 171.84139 | 324.7035 |
| PEN | 0.1571959 | 0.1724135 | 0.1459119 | 0.2457611 | 0.1391096 | 0.1992065 | 0.1683088 | 0.182543 | 0.1423281 | 0.2385819 |
| PpP | 1197.7212 | 1645.6477 | 1475.2363 | 1136.2789 | 1535.4112 | 1359.5421 | 1605.4357 | 1237.2136 | 1207.3612 | 1360.9731 |

| Monthly age | 52.27 | 54.08 | 57.19 | 57.26 | 57.26 | 62.05 | 65.3 | 74.02 | 83.08 |
|---|---|---|---|---|---|---|---|---|---|
| PYD | 164.51673 | 263.59636 | 27317743 | 229.81522 | 361.93124 | 358.36803 | 318.77232 | 198.80124 | 227.5285 |
| PEN | 01445437 | 0.1973563 | 0.1854388 | 0.199949 | 0.2944264 | 0.1802574 | 0.4271247 | 0.1568655 | 0.2489951 |
| PpP | 1138.1801 | 1335.6367 | 1473.1407 | 1149.3694 | 1229.2758 | 1988.0903 | 746.32149 | 1267.336 | 913.78694 |

A PYD being a quantification value of pyridinoline content (ng/mg), and a PEN being a quantification value of pentosidine content (ng/mg), which were quantified with a high-performance liquid chromatography with a fluorescence detector (HPLC-Flu) using heptafluorobutyric acid and formic acid as an ion-pairing reagent and were contained in a collagen-rich organic composition derived from dentin of teeth of 17 cases of TOKACHIWAKAUSHI (a Japan-registered trademark), as well as a PpP (pyridinoline content (ng/mg)/ pentosidine content (ng/mg)) being a value of a weight ratio calculated from a PYD being a quantification value of pyridinoline content (ng/mg) and a PEN being a quantification value of pentosidine content (ng/mg), are shown in Table 4 shown below.

**[Table 4]**

| Monthly age | 14 | 14 | 14 | 14 | 14 | 14 | 14 | 14 | 14 |
|---|---|---|---|---|---|---|---|---|---|
| PYD | 280.45117 | 264.98557 | 232.04947 | 255.78466 | 306.93603 | 242.9595 | 270.04606 | 262.26312 | 314.50479 |
| PEN | 0.0378339 | 0.0407724 | 0.0413905 | 0.044846 | 0.0451142 | 0.0474969 | 0.0547462 | 0.0549988 | 0.0560794 |
| PpP | 7412.7042 | 6499.1368 | 5606.3516 | 5703.6223 | 6803.5339 | 5115.2668 | 4932.6913 | 4768.5204 | 5608.2021 |

| Monthly age | 14 | 14 | 14 | 14 | 14 | 14 | 14 | 14 |
|---|---|---|---|---|---|---|---|---|
| PYD | 221.67662 | 239.64 | 261.80095 | 270.75699 | 319.92288 | 239.43516 | 313.8611 | 304.13855 |
| PEN | 0.0575293 | 0.0585127 | 0.0808979 | 0.089185 | 0.089461 | 0.0964382 | 0.1387326 | 0.1902081 |
| PpP | 3853.2832 | 4095.5216 | 3236.1887 | 3035.9024 | 3576.1161 | 2482.7846 | 2262.3457 | 1598.9776 |

A PYD being a quantification value of pyridinoline content (ng/mg), and a PEN being a quantification value of pentosidine content (ng/mg), which were quantified with a high-performance liquid chromatography with a fluorescence detector (HPLC-Flu) using heptafluorobutyric acid and formic acid as an ion-pairing reagent and were contained in a collagen-rich organic composition derived from dentin of teeth of 8 cases of livestock swine, as well as a PpP (pyridinoline content (ng/mg)/ pentosidine content (ng/mg)) being a value of a weight ratio calculated from a PYD being a quantification value of pyridinoline content (ng/mg) and a PEN being a quantification value of pentosidine content (ng/mg), are shown in Table 5 shown below.

**[Table 5]**

| Monthly age | 6 | 6 | 6 | 6 | 6 | 6 | 6 |
|---|---|---|---|---|---|---|---|
| PYD | 120.23952 | 133.55988 | 105.48836 | 109.98057 | 115.50632 | 129.9683 | 129.46338 |
| PEN | 0.0294807 | 0.0299355 | 0.0309111 | 0.0326369 | 0.0340013 | 0.0370059 | 0.0401194 |
| PpP | 4078.5857 | 4461.593 | 3412.6361 | 3369.819 | 3397.1177 | 3512.0944 | 3226.9511 |

Additionally, a distribution of a PYD being a quantification value of pyridinoline content (ng/mg) of collagen contained in a collagen-rich organic composition derived from dentin of 62 cases of teeth in total, of 18 cases of humans, 36 cases of bovines (therein, 19 cases of bovine SRM, 17 cases of TOKACHIWAKAUSHI (a Japan-registered trademark)), and 8 cases of livestock swine, and through a high-performance liquid chromatography with a fluorescence detector (HPLC-Flu) using heptafluorobutyric acid and formic acid as an ion-pairing reagent, is shown in Fig.1, whereas a distribution of a PYD being a quantification value of pyridinoline content (ng/mg) obtained by quantifying, with a high-performance liquid chromatography with a fluorescence detector (HPLC-Flu) using heptafluorobutyric acid and formic acid as an ion-pairing reagent, collagen (n=62) contained in a collagen-rich organic composition derived from dentin of teeth of 18 cases of humans, 36 cases of bovines (therein, 19 cases of bovine SRM, 17 cases of TOKACHIWAKAUSHI (a Japan-registered trademark)), and 8 cases of livestock swine, soft tissue-derived Teruplug (n=3, bovine dermis-derived), high-grade gelatin (n=3), TOKACHIWAKAWUSHI (a Japan-registered trademark)'s gums (n=3), and TOKACHIWAKAWUSHI (a Japan-registered trademark)'s muscles (n=3) is shown in Fig.2.

A PYD being a quantification value of pyridinoline content (ng/mg) was, as shown in Fig.1, included in a PYD scope according to the present invention in any way for collagen contained in a collagen-rich organic composition derived from dentin of teeth (62 cases in total) being one example of a hard tissue of 18 cases of humans, being a mammal, as well as 36 cases of bovines (therein, 19 cases of bovine SRM, 17 cases of TOKACHIWAKAUSHI (a Japan-registered trademark)), and 8 cases of livestock swine, being a mammal and a livestock animal simultaneously.

On the other hand, a PYD being a quantification value of pyridinoline content (ng/mg) was, as shown in Fig.2, 50 or lower in any way for soft tissue-derived Teruplug (n=3, bovine dermis-derived), high-grade gelatin (n=3), TOKACHIWAKAWUSHI (a Japan-registered trademark)'s gums (n=3), and TOKACHIWAKAWUSHI (a Japan-registered trademark)'s muscles (n=3).

From the above, a PYD value according to the present invention was set to PYD>100 in view of a lower limit value of a PYD of collagen contained in a collagen-rich organic composition derived from dentin of teeth being a hard tissue of 18 cases of humans, being a mammal, as well as 36 cases of bovines and 8 cases of livestock swine, being a mammal and a livestock animal simultaneously.

Additionally, a distribution of a PEN being a quantification value of pentosidine content (ng/mg), obtained by quantifying, with a high-performance liquid chromatography with a fluorescence detector (HPLC-Flu) using heptafluorobutyric acid and formic acid as an ion-pairing reagent, collagen contained in a collagen-rich organic composition derived from dentin of 62 cases of teeth in total, of 18 cases of humans, 36 cases of bovines (therein, 19 cases of bovine SRM, 17 cases of TOKACHIWAKAUSHI (a Japan-registered trademark)), and 8 cases of livestock swine is shown in Fig.3.

A PEN being a quantification value of pentosidine content (ng/mg) of collagen contained in a collagen-rich organic composition derived from dentin of teeth being one example of a hard tissue of 44 cases of livestock animals (therein, 19 cases of bovine SRM, 17 cases of TOKACHIWAKAWUSHI (a Japan-registered trademark), and 8 cases of livestock swine) was, as shown in Fig.3, set to 0<PEN<0.4 in view of upper limit values of 44 cases of livestock animals.

Additionally, a value distribution of a PpP (pyridinoline content (ng/mg)/ pentosidine content (ng/mg)) being a value of a weight ratio calculated from a PYD being a quantification value of pyridinoline content (ng/mg) and a PEN being a quantification value of pentosidine content (ng/mg), which are obtained by quantifying, with a high-performance liquid chromatography with a fluorescence detector (HPLC-Flu) using heptafluorobutyric acid and formic acid as an ion-pairing reagent, collagen contained in a collagen-rich organic composition derived from dentin of 62 cases of teeth in total, of 18 cases of humans, 36 cases of bovines (therein, 19 cases of bovine SRM, 17 cases of TOKACHIWAKAUSHI (a Japan-registered trademark)), and 8 cases of livestock swine is shown in Fig.4.

A PpP (pyridinoline content (ng/mg)/ pentosidine content (ng/mg)) being a value of a weight ratio between a PYD being a quantification value of pyridinoline content (ng/mg) and a PEN being a quantification value of pentosidine content (ng/mg) was, as shown in Fig.4, set to PpP>740 since livestock animals are made to be indicators.

### <Embodiment 2> An endotoxin test for collagen derived from bovine teeth (mainly, dentin), porcine skin-derived gelatin, and high-grade gelatin

An endotoxin test was carried out for collagen derived from teeth (mainly, dentin) of 6 cases of TOKACHIWAKAUSHI (a male Holstein with a Japan-registered trademark and through early fattening (14 months old); JA Tokachi Shimizu), and for 3 cases of porcine skin-derived gelatin (Merck), and 3 cases of high-grade gelatin. The endotoxin test was carried out by using, in a colorimetric method, an LAL reagent made from a limulus amebocyte lysate obtained by using horseshoe crab-derived blood. A result thereof is shown in Fig.5.

A sample of collagen derived from teeth of 6 cases of TOKACHIWAKAUSHI (a Japan-registered trademark) was prepared in a method below.
(1) As for collagen whose mineral content remained, a decalcification solution containing 500mM of EDTA was used and the decalcification solution was, while being replaced, being shaken at 37°C for 2 days to remove, thereby, the mineral content completely, and then, the EDTA was removed by being washed away by ultrapure water.
(2) Collagen obtained was completely dried and dried collagen was obtained thereby.
(3) The dried collagen was completely decomposed by using Proteinase K (Takara Bio Inc.) under a condition of 56°C.
(4) Then, the Proteinase K was, by being heated at 95°C for 5 minutes, made to lose its activity.
(5) Centrifugation was made at 12000 rpm under room temperature to make a supernatant obtained a collagen sample.

As shown in Fig.5, an endotoxin level was less than 0.125 for collagen derived from teeth of 6 cases of TOKACHIWAKAUSHI (a Japan-registered trademark), the endotoxin level was around 1.25 for 3 cases of porcine skin-derived gelatin, and the endotoxin level was less than 0.125 for 3 cases of high-grade gelatin.

As seen from a fact that an endotoxin value of standard is 15 EU/mg for a famotidine injection, the endotoxin value of standard is 6.0 EU/mg for thiamine chloride hydrochloride injection, the endotoxin value of standard is 4.0 EU/mg for injection-used roxatidine acetate ester hydrochloride, the endotoxin value of standard is 3.0 EU/mg for pyridoxine hydrochloride injection, and that the endotoxin value of standard is 1.5 EU/mg for morphine hydrochloride injection, an endotoxin level of collagen derived from teeth of 6 cases of TOKACHIWAKAUSHI (a Japan-registered trademark) and an endotoxin level of 3 cases of high-grade gelatin are, needless to say, extremely excellent, and even an endotoxin level of 3 cases of porcine skin-derived gelatin can be said to be excellent.

That is, it has been made clear that since a collagen-rich organic composition according to the present application invention has its endotoxin contained substantially reduced in quantity, or its endotoxin contained substantially reduced in activity, it is substantially free of endotoxin or free of endotoxin, or alternatively has its endotoxin contained substantially inactivated or such endotoxin inactivated.

## Claims

1. A collagen-rich organic composition produced from teeth or bones extracted from a mammal, of which (i) or (i) and (ii) below holds:
(i) As for a PYD being a quantification value of pyridinoline content (ng/mg) of collagen which is quantified with a high-performance liquid chromatography with a fluorescence detector (HPLC-Flu) using heptafluorobutyric acid and formic acid as an ion-pairing reagent and is contained in the collagen-rich organic composition, PYD>100,
(ii) As for a PpP (pyridinoline content (ng/mg)/ pentosidine content (ng/mg)) being a value of a weight ratio between a PYD being a quantification value of pyridinoline content (ng/mg) and a PEN being a quantification value of pentosidine content (ng/mg), of collagen which is quantified with a high-performance liquid chromatography with a fluorescence detector (HPLC-Flu) using heptafluorobutyric acid and formic acid as an ion-pairing reagent and is contained in the collagen-rich organic composition, PpP>740.

2. A collagen-rich organic composition according to Claim 1, which is substantially free of endotoxin or free of endotoxin, or alternatively has its endotoxin contained substantially inactivated or such endotoxin inactivated.

3. A collagen-rich organic composition according to Claim 1, wherein the mammals are 1 or 2 or more mammals to be selected from a group consisting of bovines, swine, horses, sheep, deer, dogs, cats, and humans.

4. A biomaterial or a biomaterial or other product containing collagen as a main ingredient, which uses a collagen-rich organic composition according to one claim of Claim 1 to Claim 3 for an ingredient material.

5. A method of producing a collagen-rich organic composition according to one claim of Claim 1 to Claim 3, which has
a step of obtaining a pulverization product by pulverizing teeth or bones extracted from a mammal,
a step of giving strong alkali treatment to the pulverization product obtained,
and a step of giving demineralization treatment under a negative pressure condition to the pulverization product given strong alkali treatment.

6. A production method according to Claim 5, wherein the step of giving strong alkali treatment to the pulverization product obtained is a step of giving strong alkali treatment under a condition of temperature higher than normal temperature and lower than water boiling point to the pulverization product obtained.

7. A method of discerning teeth or bones extracted from a mammal to be used as an ingredient material of a biomaterial or a biomaterial or other product containing collagen as a main ingredient mammal, which has
a step of quantifying pyridinoline content (ng/mg) of collagen contained in teeth or bones extracted from a mammal,
a step of quantifying pentosidine content (ng/mg) of the collagen contained in teeth or bones extracted from a mammal,
a step of calculating, from a PYD being a value of the pyridinoline content (ng/mg) quantified and a PEN being a value of the pentosidine content (ng/mg) quantified, a PpP (pyridinoline content (ng/mg)/ pentosidine content (ng/mg)) being a weight ratio,
and a step of selecting, when a PYD being a value of the pyridinoline content (ng/mg) quantified or a PYD being a value of the pyridinoline content (ng/mg) quantified and a PpP (pyridinoline content (ng/mg)/ pentosidine content (ng/mg)) being a value of the weight ratio calculated are specified values, the teeth or bones extracted from a mammal for an ingredient material of a biomaterial or a biomaterial or other product containing collagen as a main ingredient.

8. A method of discerning teeth or bones extracted from a mammal to be used as an ingredient material of a biomaterial or a biomaterial or other product containing collagen as a main ingredient mammal, which has
a step of obtaining a pulverization product by pulverizing teeth or bones extracted from a mammal,
a step of giving strong alkali treatment to the pulverization product obtained,
a step of giving demineralization treatment under a negative pressure condition to the pulverization product given strong alkali treatment, to obtain a collagen-rich organic composition,
a step of quantifying pyridinoline content (ng/mg) of collagen contained in the collagen-rich organic composition obtained,
a step of quantifying pentosidine content (ng/mg) of collagen contained in the collagen-rich organic composition obtained,
a step of calculating, from a PYD being a value of the pyridinoline content (ng/mg) quantified and a PEN being a value of the pentosidine content (ng/mg) quantified, a PpP (pyridinoline content (ng/mg)/ pentosidine content (ng/mg)) being a value of a weight ratio,
and a step of selecting, when a PYD being a value of the pyridinoline content (ng/mg) quantified or a PYD being a value of the pyridinoline content (ng/mg) quantified and a PpP (pyridinoline content (ng/mg)/ pentosidine content (ng/mg)) being a value of the weight ratio calculated are specified values, the teeth or bones extracted from a mammal for an ingredient material of a biomaterial or a biomaterial or other product containing collagen as a main ingredient.

9. A method according to Claim 7 or Claim 8, wherein the step of quantifying pyridinoline content (ng/mg) of collagen contained in the collagen-rich organic composition obtained and the step of quantifying pentosidine content (ng/mg) of collagen contained in the collagen-rich organic composition obtained are a step of quantifying, with a high-performance liquid chromatography with a fluorescence detector (HPLC-Flu) using heptafluorobutyric acid and formic acid as an ion-pairing reagent, pyridinoline content (ng/mg) of collagen contained in the collagen-rich organic composition obtained and a step of quantifying, therewith, pentosidine content (ng/mg) of collagen contained in the collagen-rich organic composition obtained, and wherein as for specified values, PYD>100 or PYD>100 and PpP>740, respectively.

10. A method of using, as an indicator for assessing a potential morbidity risk of a disease in a mammal and/or whether a mammal is potentially in good health or not, a quantification value of pyridinoline content of collagen contained in teeth of a mammal, or a quantification value of pyridinoline content of collagen contained in teeth of a mammal as well as a weight ratio calculated from a quantification value of pyridinoline content of collagen contained in teeth of the mammal and a quantification value of pentosidine content thereof, which includes
a step of obtaining, by quantifying pyridinoline content (ng/mg) of collagen contained in teeth of a mammal to be assessed for a potential morbidity risk of a disease and/or whether a mammal is potentially in good health or not, a PYD being a quantification value,
a step of obtaining, by quantifying pentosidine content (ng/mg) of the collagen contained in teeth of a mammal to be assessed, a PEN being a quantification value,
a step of calculating, from the PYD obtained and the PEN obtained, a PpP (pyridinoline content (ng/mg)/ pentosidine content (ng/mg)) being a value of a weight ratio,
a step of obtaining, by quantifying pyridinoline content (ng/mg) of collagen contained in teeth of 1 or 2 or more mammals selected from a mammal not affected by a disease, a mammal affected by a disease, a mammal in good health, and a mammal not in good health, a R-PYD being a reference quantification value,
a step of obtaining, by quantifying pentosidine content (ng/mg) of collagen contained in teeth of 1 or 2 or more mammals selected from a mammal not affected by a disease, a mammal affected by a disease, a mammal in good health, and a mammal not in good health, a R-PEN being a reference quantification value,
a step of calculating, from the R-PYD obtained and the R-PEN obtained, a R-PpP (pyridinoline content (ng/mg)/ pentosidine content (ng/mg)) being a reference value of a weight ratio,
and a step of comparing the PYD and the R-PYD or comparing the PYD and the R-PYD as well as the PpP and the R-PpP.

11. A method according to Claim 10, wherein the step of obtaining, by quantifying pyridinoline content (ng/mg), a R-PYD being a reference quantification value and the step of obtaining, by quantifying pentosidine content (ng/mg), a R-PEN being a reference quantification value are a step of quantifying, with a high-performance liquid chromatography with a fluorescence detector (HPLC-Flu) using heptafluorobutyric acid and formic acid as an ion-pairing reagent, the pyridinoline content (ng/mg) to obtain a R-PYD being a reference value and a step of quantifying, therewith, the pentosidine content (ng/mg) to obtain a R-PEN being a reference value, and where a step of comparing the PYD and the R-PYD or comparing the PYD and the R-PYD as well as comparing the PpP and the R-PpP is a step of checking whether or not, as for the PYD, PYD>100 or checking whether or not, as for the PYD, PYD>100 and whether or not, as for the PpP, PpP>740.
